# EUROPEAN PATENT SPECIFICATION

(11) **EP 4 314 010 B1**
(45) Date of publication and mention of the grant of the patent: **11.11.2026**
(21) Application number: 22776504.7
(22) Date of filing: 22.03.2022
(51) Int. Cl.: C07K 7/06, C07K 7/08, A61K 38/00, A61P 11/06, A61P 31/04, A61P 31/10, A61P 31/14

(54) **MODIFIED PEPTIDOMIMETICS AND METHODS OF USE**
MODIFIZIERTE PEPTIDOMIMETIKA UND VERFAHREN ZUR VERWENDUNG
PEPTIDOMIMÉTIQUES MODIFIÉS ET PROCÉDÉS D'UTILISATION

(30) Priority: 22.03.2021 US 202163164132 P
(43) Date of publication of application: 07.02.2024
(73) Proprietor: The University of North Carolina at Chapel Hill, Chapel Hill, NC 27514 (US)
(72) Inventor: TARRAN, Robert, Chapel Hill, North Carolina 27516 (US); SASSANO, Maria Florencia, Morrisville, North Carolina 27560 (US); AHMAD, Saira, Chapel Hill, North Carolina 27514 (US)
(74) Representative: V.O.
(86) International application number: PCT/US2022/021391
(87) International publication number: WO 2022/204185

(56) References cited:
- WO-A1-2013/043720
- WO-A1-2017/147128
- CN-A- 106 749 679
- US-A1- 2019 062 386
- TONGDE WU ET AL: "Identification of BPIFA1/SPLUNC1 as an epithelium-derived smooth muscle relaxing factor", NATURE COMMUNICATIONS, vol. 8, 14118, 26 February 2017 (2017-02-26), UK, XP055622448, ISSN: 2041-1723, DOI: 10.1038/ncomms14118
- C. A. HOBBS ET AL: "Identification of the SPLUNC1 ENaC-inhibitory domain yields novel strategies to treat sodium hyperabsorption in cystic fibrosis airway epithelial cultures", AMERICAN JOURNAL OF PHYSIOLOGY - LUNG CELLULAR AND MOLECULAR PHYSIOLOGY, vol. 305, no. 12, 15 December 2013 (2013-12-15), US, pages 990 - 1001, XP055411764, ISSN: 1040-0605, DOI: 10.1152/ajplung.00103.2013
- TONGDE WU, JULIANNE HUANG, PATRICK J. MOORE, MICHAEL S. LITTLE, WILLIAM G. WALTON, ROBERT C. FELLNER, NEIL E. ALEXIS, Y. PETER DI,: "Identification of BPIFA1/SPLUNC1 as an epithelium-derived smooth muscle relaxing factor", NATURE COMMUNICATIONS, NATURE PUBLISHING GROUP, UK, vol. 8, no. 1, 1 April 2017 (2017-04-01), UK, XP055622448, ISSN: 2041-1723, DOI: 10.1038/ncomms14118
- DATABASE UNIPROTKB 1 October 2000 (2000-10-01), ANONYMOUS : "Q9NP55 (BPIA1_HUMAN)", XP055969233, retrieved from UNIPROT Database accession no. Q9NP55

## Description

### FIELD OF THE INVENTION

This invention relates to synthetic polypeptides that bind to the Orai1 calcium channel, and their therapeutic use as lung immunomodulators in disorders such as, but not limited to, viral infections, bacterial infections, allergic responses, asthma, cystic fibrosis and other inflammatory disorders.

### BACKGROUND OF THE INVENTION

Orai1 is a ubiquitously-expressed plasma membrane Ca²⁺ channel, and an essential, proximal component of the immune system/inflammatory cascade. Orai1 is tightly regulated, since too much Orai1 contributes to hyperinflammation, while body-wide inhibition of Orai1 is immunosuppressive. Thus, there is a need for fine-tuned, local modulation of Orai1 function.

The present invention overcomes previous shortcomings by providing synthetic polypeptides that bind to the Orai1 calcium channel and reduce its expression and functionality specifically in the lungs while leaving normal systemic Orai1 functioning unaffected, as well as providing methods of using these synthetic polypeptides to treat disorders responsive to modulating Orai1.

Further background art includes Tongde Wu et al., Nature Communications, vol. 8, Article 14118, 26 February 2017, pages 1-10; WO2017/147128A1; and C. A. Hobbs et al. American Journal of Physiology - Lung Cellular and Molecular Physiology, vol. 305, no. 12, 15 December 2013, pages 990-1001.

### SUMMARY OF THE INVENTION

The invention is set out in the appended set of claims.

These and other aspects of the invention are set forth in more detail in the description of the invention below.

### BRIEF DESCRIPTION OF THE DRAWINGS

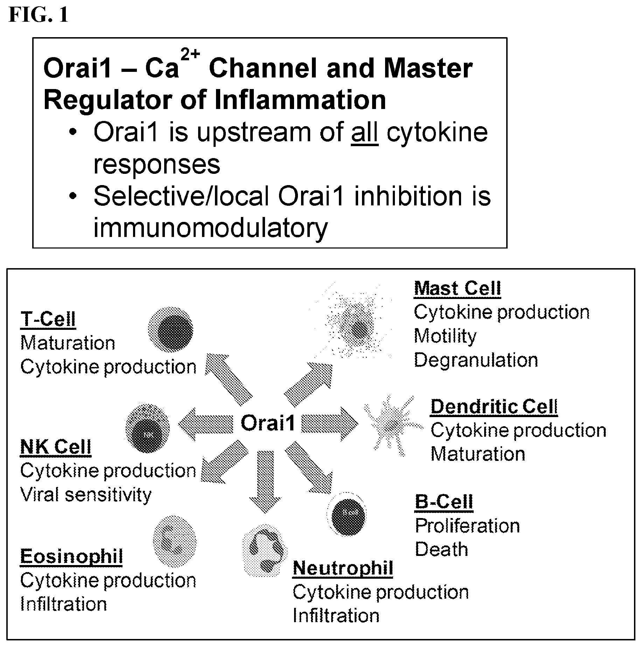

**FIG. 1** shows a schematic of cell types influenced by Orai1.

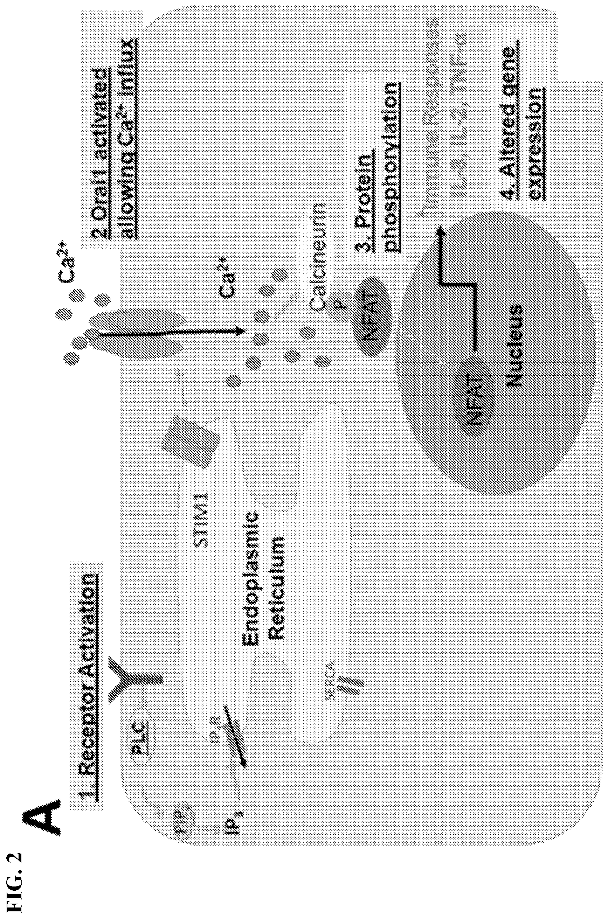

**FIG. 2** shows a schematic of Orai1 related signaling. During infection, increased SOCE and Orai1 activity leads to greater phosphorylation of Ca²⁺-sensitive kinases (e.g., calcineurin) and activation of transcription factors (e.g., NFAT) leading to a pro-inflammatory response. ELD607 binds to and internalizes Orai1, preventing SOCE, despite ongoing upstream receptor engagement.

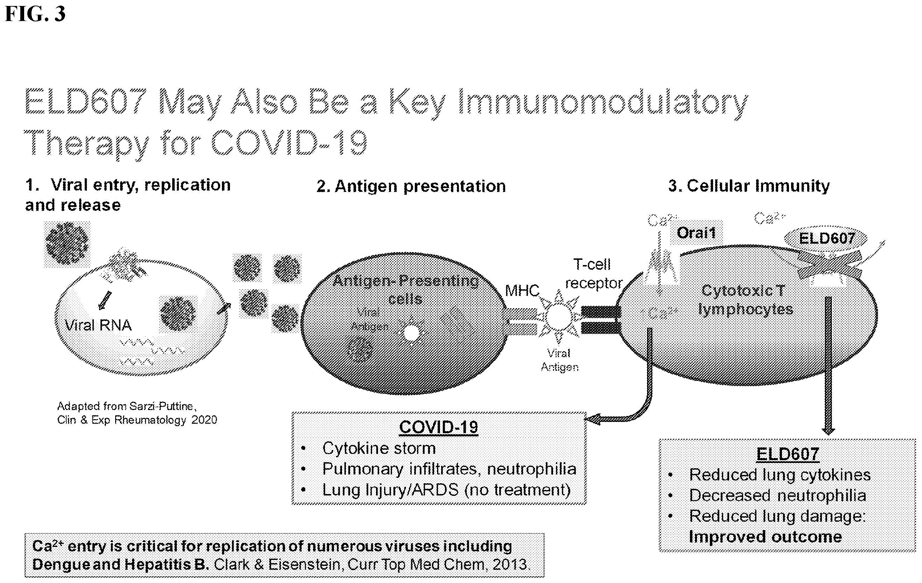

**FIG. 3** shows a schematic of ELD607 inhibitory activity on Orai1 as related to SARS-CoV-2 infection (COVID-19).

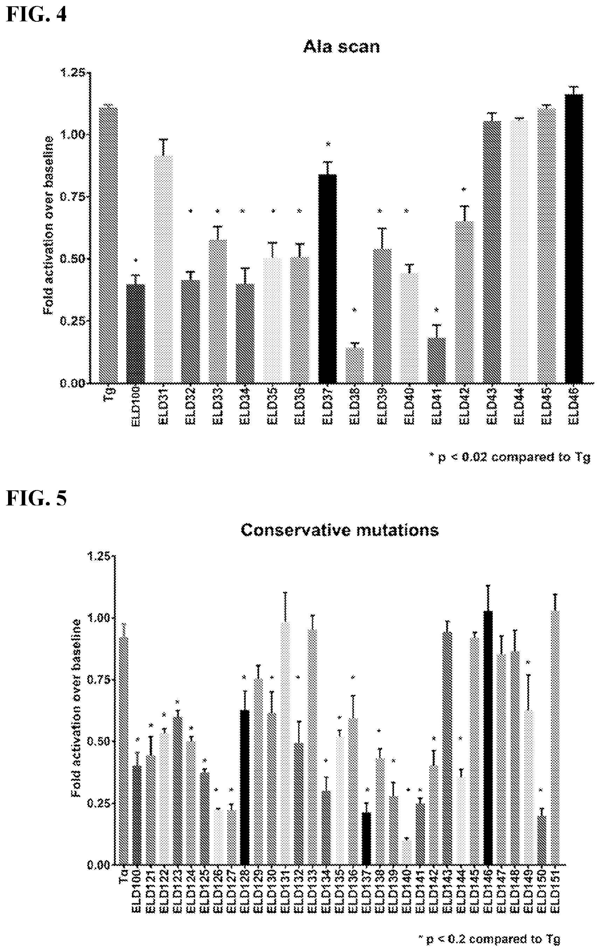

**FIG. 4** shows a bar graph of Ca²⁺ levels for Alanine scan peptides after thapsigargin addition. The results were normalized to the baseline. Thapsigargin (Tg) at 1 µM was used as control. The inhibition of Ca²⁺ release after scaffold peptide (ELD100; reference) addition is shown for comparison. N=9. * p < 0.02.
**FIG. 5** shows a bar graph of Ca²⁺ levels for peptides that underwent conservative mutations upon thapsigargin addition. The results were normalized to the baseline. Thapsigargin (Tg) at 1 µM was used as control. The inhibition of Ca²⁺ release after ELD100 (reference) addition is shown for comparison. N=9. * p < 0.02.

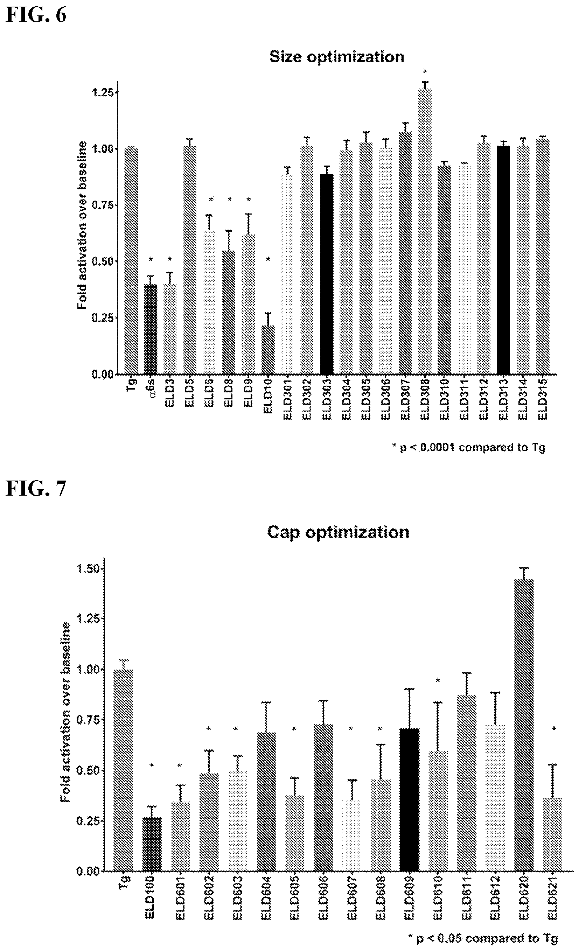

**FIG. 6** shows a bar graph of Ca²⁺ levels upon thapsigargin addition for peptides that were size-optimized. The results were normalized to the baseline. Thapsigargin (Tg) at 1 µM was used as control. The inhibition of Ca²⁺ release after ELD100 (reference) addition is shown for comparison. N=9. * p < 0.0001.
**FIG. 7** shows a bar graph of Ca²⁺ levels upon thapsigargin addition for peptides that were size-optimized and capped for stability. The results were normalized to the baseline. Thapsigargin (Tg) at 1 µM was used as control. The inhibition of Ca²⁺ release after ELD100 (reference) addition is shown for comparison. N=9. * p < 0.05.

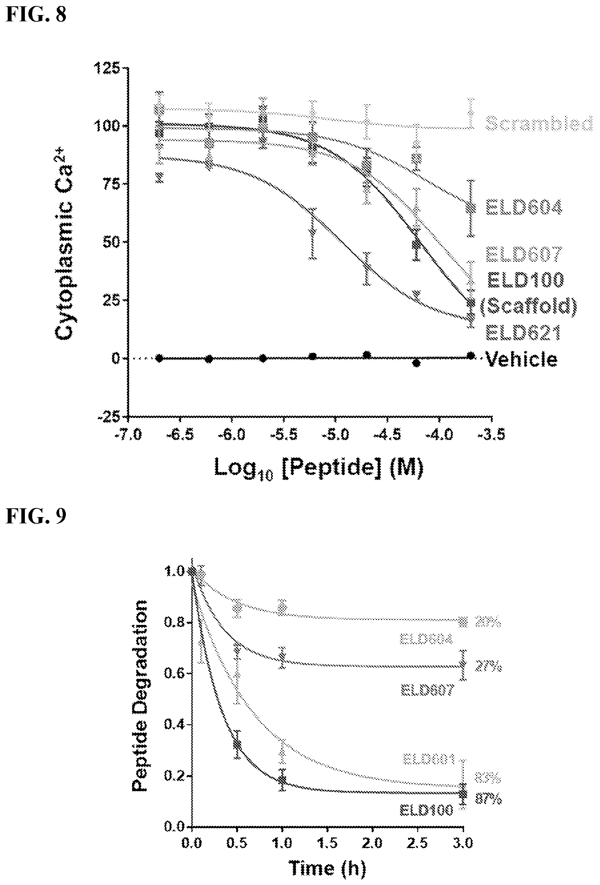

**FIG. 8** shows a graph of cytoplasmic Ca²⁺ of HEK293T cells treated with the noted peptides.
**FIG. 9** shows a graph of ELD604 (not claimed) and ELD607 resistance to neutrophil elastase-mediated degradation. Peptides were exposed to NE over time, and the reaction was stopped by addition of sivelestat. HPLC was then performed in order to determine peptide stability. N=3-4 replicates per time point.

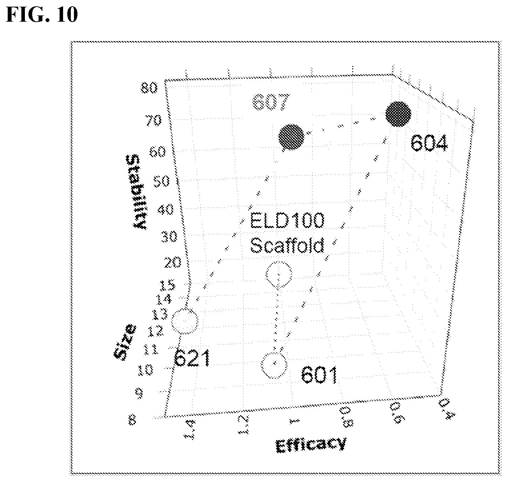

**FIG. 10** shows a 3D graph comparing elastase stability, efficacy, and size for the 600 series of compounds vs. the ELD100 scaffold.

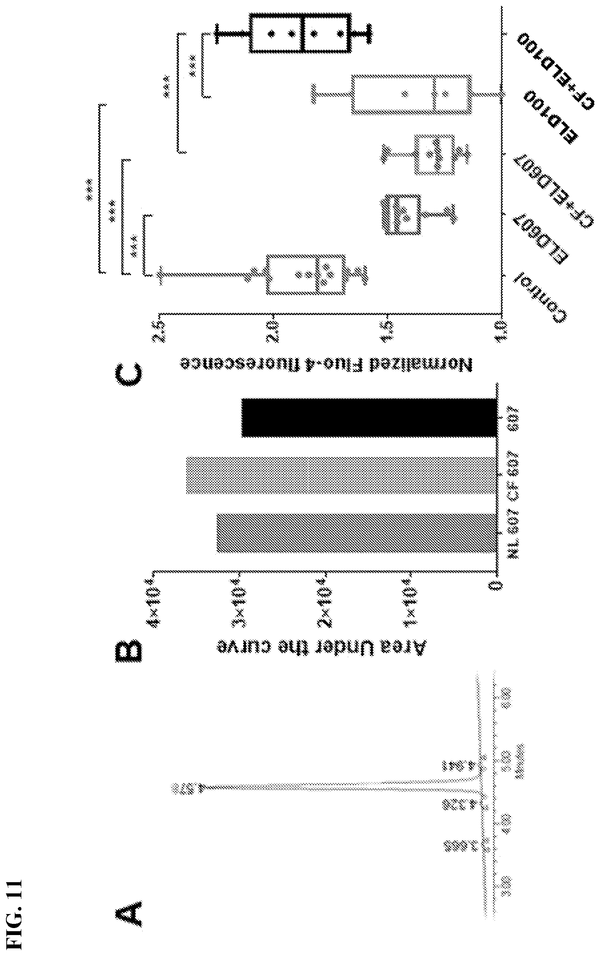

**FIG. 11** shows that ELD607 is stable in CF sputum. **FIG. 11** **panel A** shows a graph of HPLC analysis demonstrating a peak at ~4.5 minutes that corresponds to ELD607. **FIG. 11** **panel B** shows a bar graph of NL and CF sputum each pooled from 3 different donors. ELD607 was incubated in NL and CF sputum for 6 h at 37°C vs PBS (control) and samples were passed through a 10 kDa spin column to halt the reaction. The area under the curve was similar for each of the conditions. **FIG. 11** **panel C** shows a graph of ELD607 or ELD100 (reference) incubated in either PBS or CF sputum and added to HEK293T cells. 1 h later, thapsigargin was added and the change in Fluo-4 fluorescence was measured. Data show the peak Fluo-4 response. Control is addition of thapsigargin alone. *p<0.001 different to control.

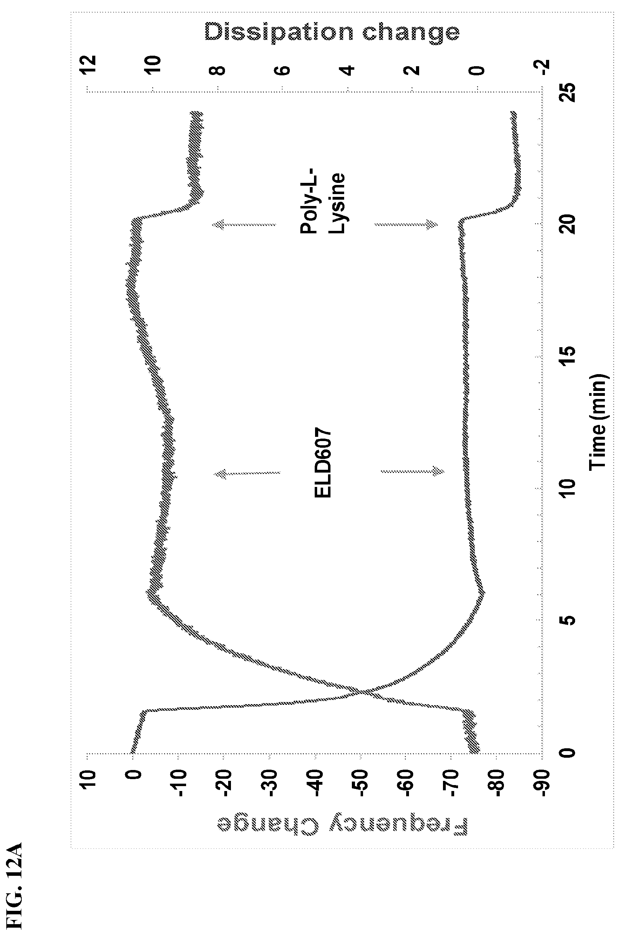

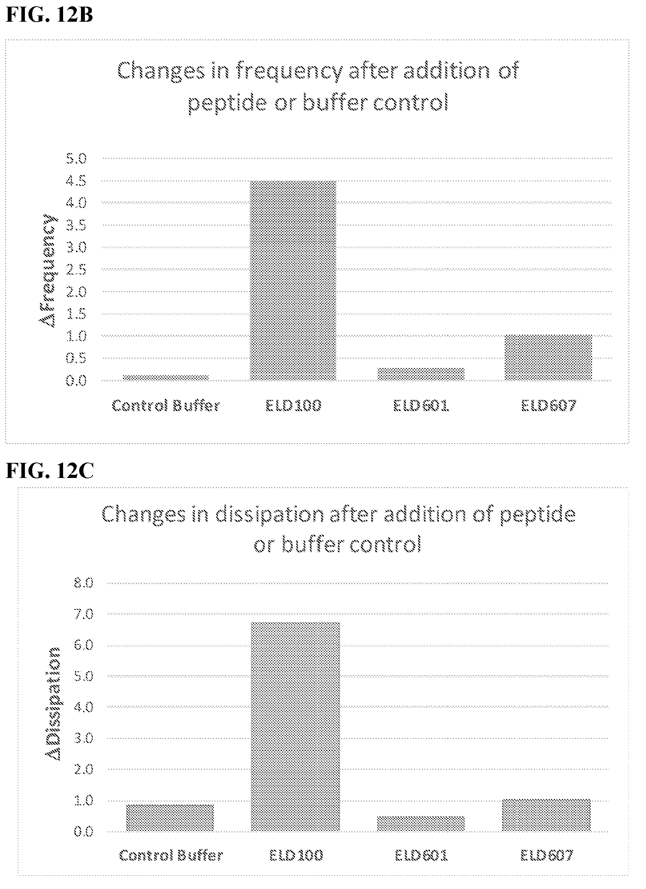

**FIG. 12A** shows a graph indicating that ELD607 does not interact with mucus. A Quartz Crystal Microbalance with Dissipation (QCM-D) assay was used to measure human airway dissipation and frequency as indicators of mucus-cross linking and mass, respectively. ELD607 did not alter these properties. In contrast, poly-L-lysine (positive control) bound to mucus, which altered cross-linking and mass. **FIG. 12B** shows a bar graph of summary data for QCM-D frequency for the peptides indicated. **FIG. 12C** shows a bar graph of summary data for QCM-D dissipation for the peptides indicated.

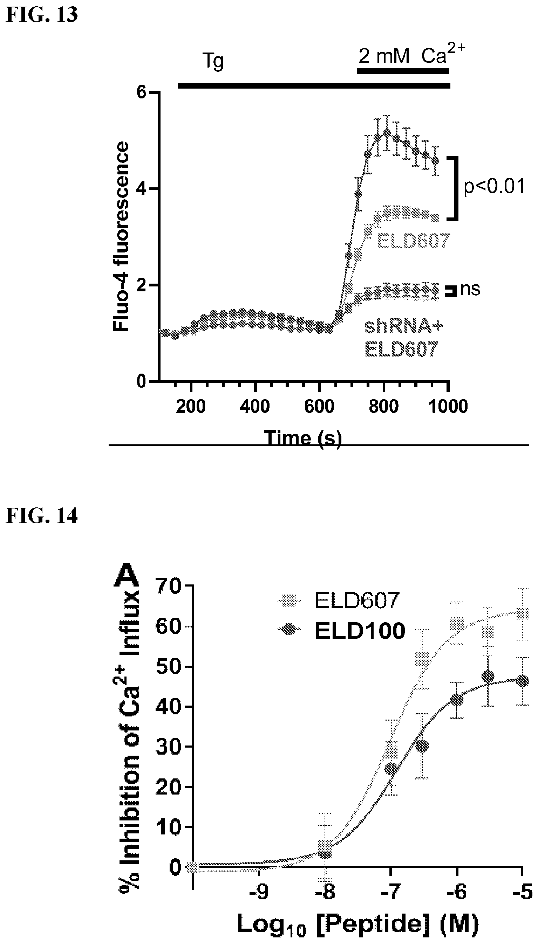

**FIG. 13** shows fluorescence measurements indicating that Orai1 knockdown prevents ELD607 from inhibiting SOCE. HEK293T cells were transfected with anti-Orai1 shRNA for 48 h and the change in Fluo4 fluorescence was measured. Cells were then exposed to vehicle or 10 mM ELD607 for 1 h. Thapsigargin was then added followed by extracellular Ca²⁺ to differentiate ER Ca²⁺ release and SOCE. n=8 cultures per condition.
**FIG. 14** shows a graph of dose response curves for ELD607 and ELD100 (reference) based on Ca2+ influx rather than global Ca²⁺. HEK293T cells were cultured in 384 well plates. Cells were then exposed to vehicle or 10 mM ELD607 for 1 h. 100 nM thapsigargin was then added followed by extracellular Ca²⁺ to differentiate ER Ca²⁺ release and SOCE. n=8-12 cultures per data point.

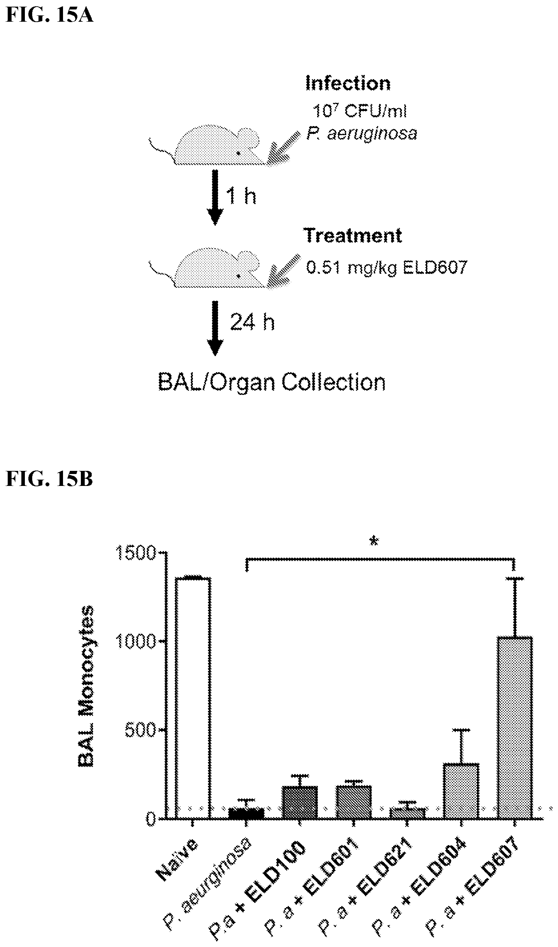

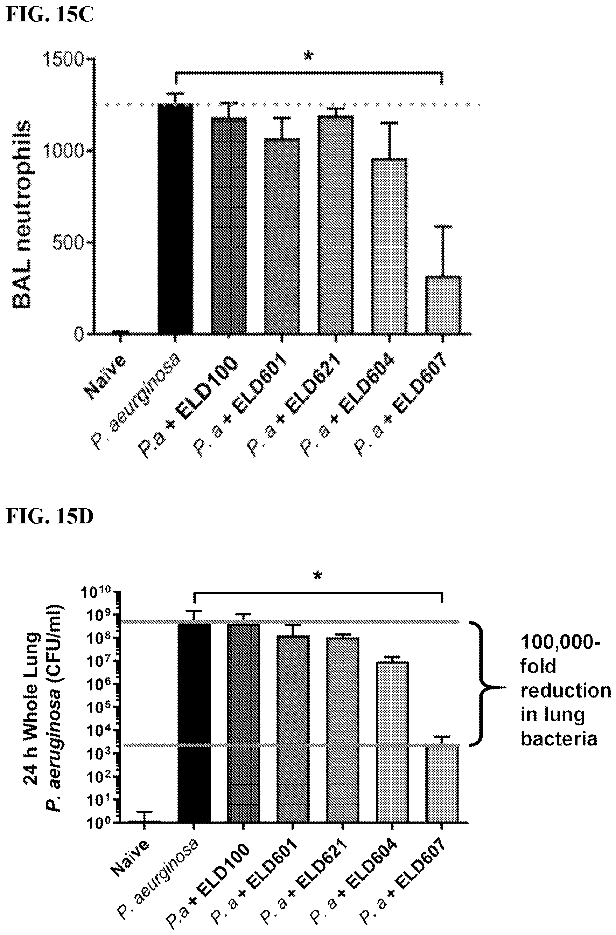

**FIGS. 15A-15D** show **(****FIG. 15A****)** a schematic of a murine model of *P. aeruginosa* (top left panel), and bar graphs of **(****FIG. 15B****)** BAL monocytes, **(****FIG. 15C****)** BAL neutrophils, and **(****FIG. 15D****)** bacterial counts in whole lung at 24 hours quantified following experiments according to the mouse model as schematized and conditions as noted on the x-axis.

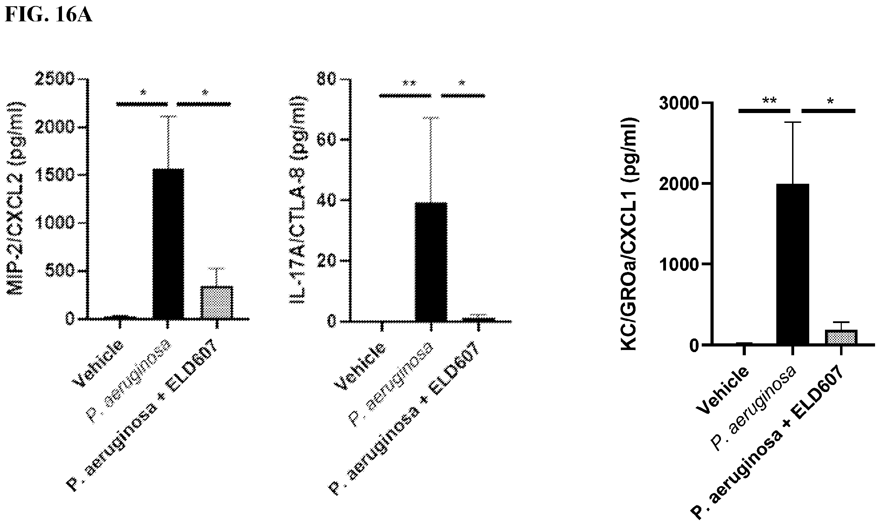

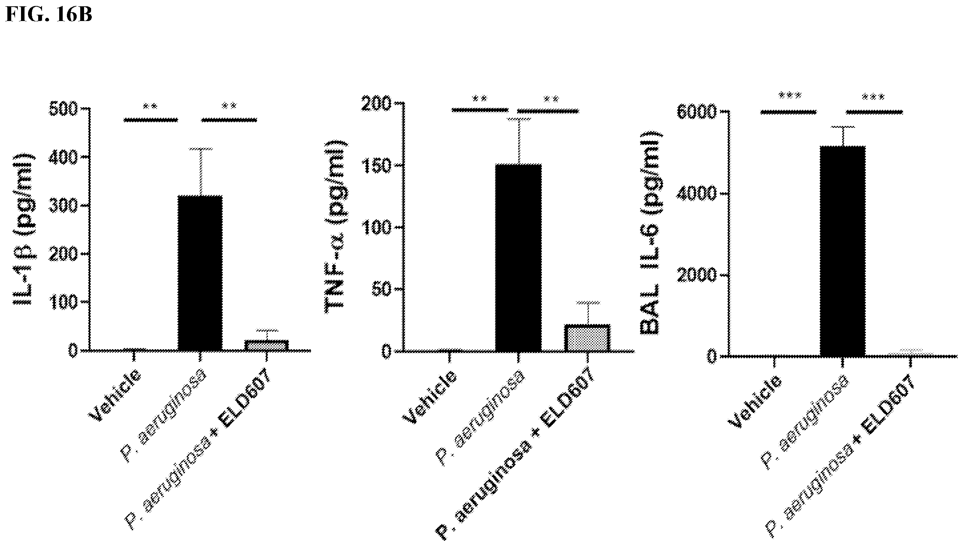

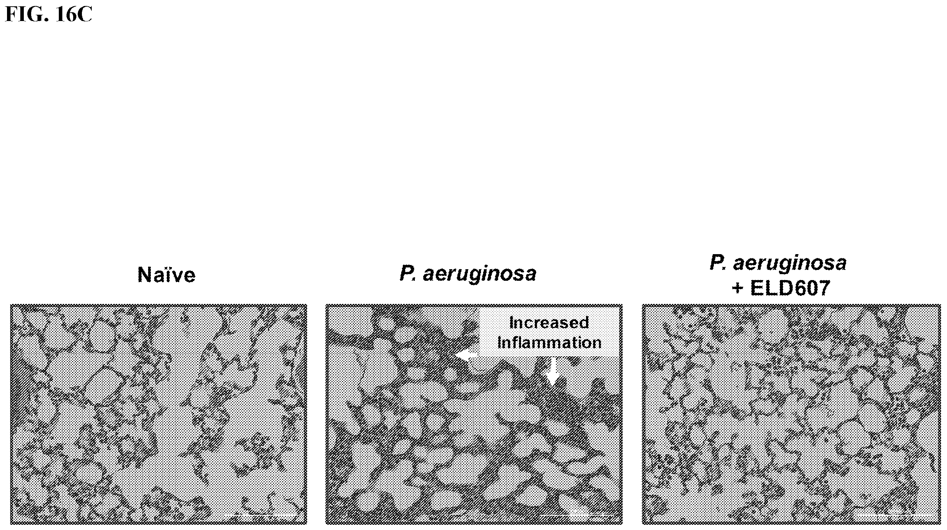

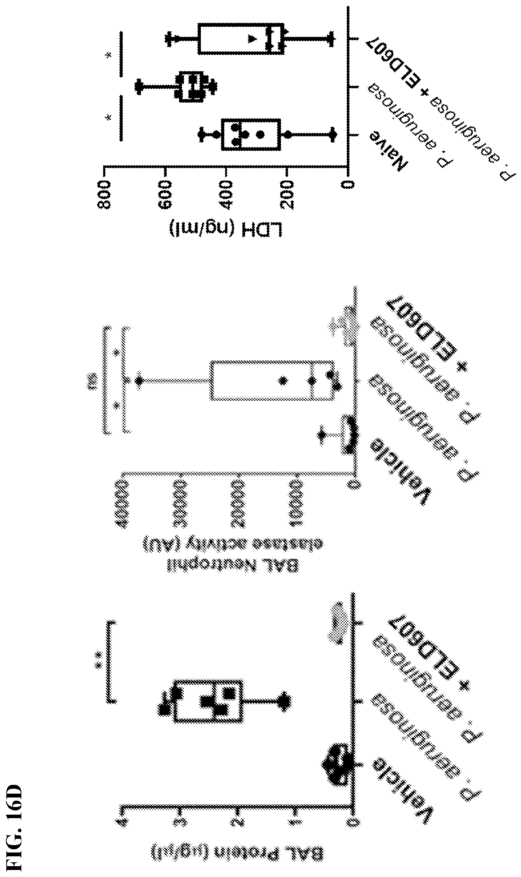

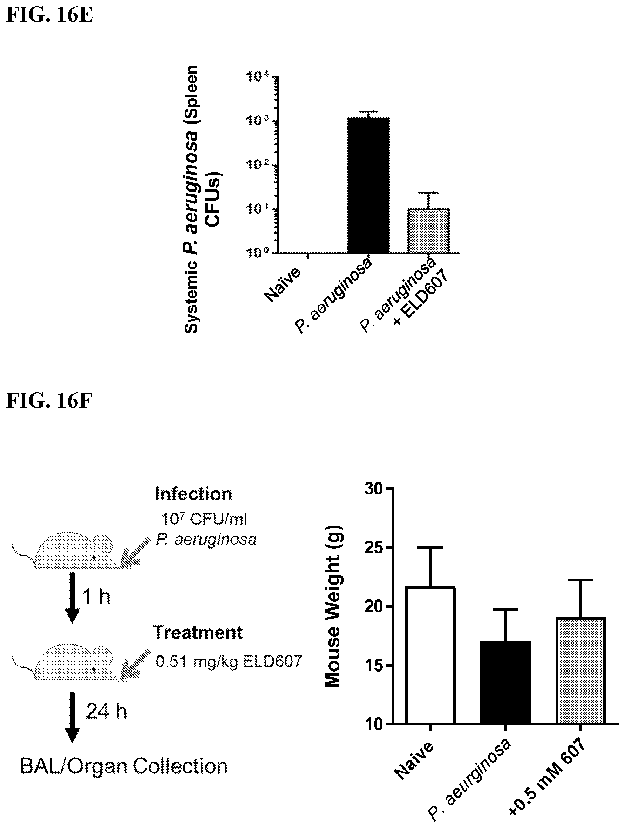

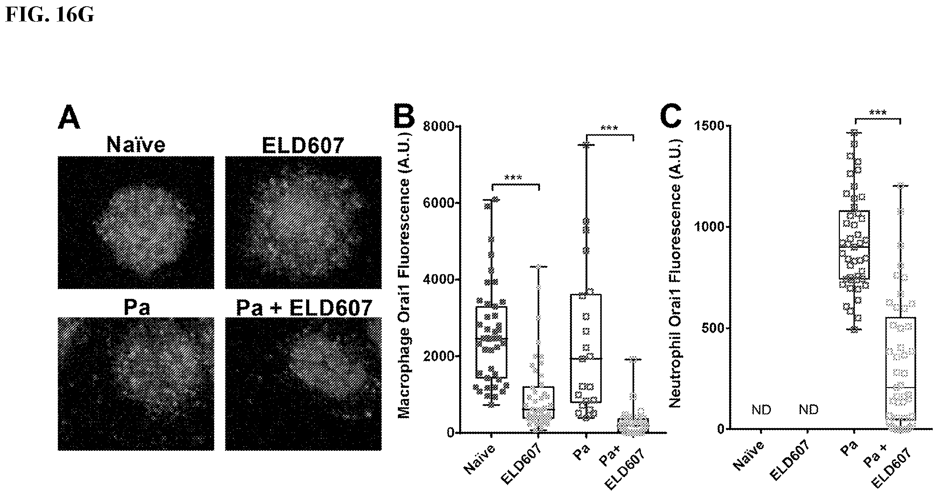

**FIGS. 16A-16G** show data from further *P. aeruginosa* infection experiments. **FIG. 16A** shows bar graphs of lung cytokine levels (left, MIP-2/CXCL2; middle, IL-17/CTLA-8; right KC/GROa/CXCL1) in the *P. aeruginosa* infection model with or without treatment of ELD607. **FIG. 16B** shows further bar graphs of lung cytokine levels (left, IL-1β; middle, TNF-α; right BAL IL-6) in the *P. aeruginosa* infection model with or without treatment of ELD607. **FIG. 16C** shows images of lung histology from mice according to the indicated conditions: naïve (left), *P. aeruginosa-infected* (middle) or *P. aeruginosa-infected* and treated with ELD607. Increased inflammation is seen in infection as indicated in the alveolar/gas exchange regions of the lungs which show alveolar damage and thickening that prevent gas exchange. ELD607 treatment normalized alveoli histology similar to naive lung conditions. **FIG 16D** shows bar graphs of total bronchoalveolar lavage (BAL) protein levels (left), total BAL neutrophil elastase levels (middle), total lactate dehydrogenase (LDH) levels (right) in the *P. aeruginosa* infection model with or without treatment of ELD607. **FIG. 16E** shows a bar graph of bacterial counts (CFU/ml) in the spleen of the *P. aeruginosa* infected mice 24 hours post ELD607 treatment. **FIG. 16F** shows a schematic of the infection protocol and a bar graph of % weight loss in the *P. aeruginosa* infected mice 24 hours post ELD607 treatment.
**FIG. 16G** shows histology and graphs of BAL analysis, suggesting that ELD607 reduces Orai1 in murine BAL macrophages. Mice were exposed to vehicle *or P. aeruginosa* ± ELD607 and BAL was obtained and stained with an anti-Orai1 antibody, fluorescent 2° and DAPI. **FIG. 16G** **panel A** shows representative images of macrophages from the different groups as indicated. **FIG. 16G** **panels B and C** show plots showing fluorescence intensities of Orai1 in macrophages and neutrophils, respectively. *** p<0.001 different as indicated. ND = no detectable neutrophils in these groups.

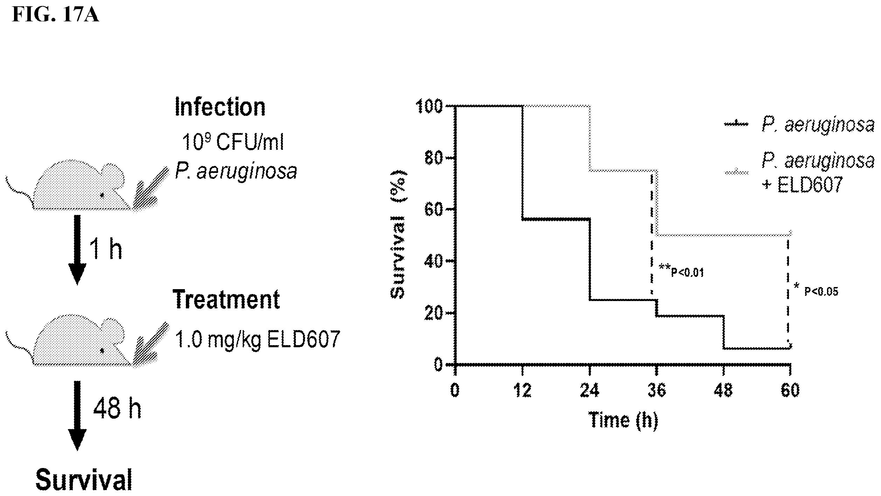

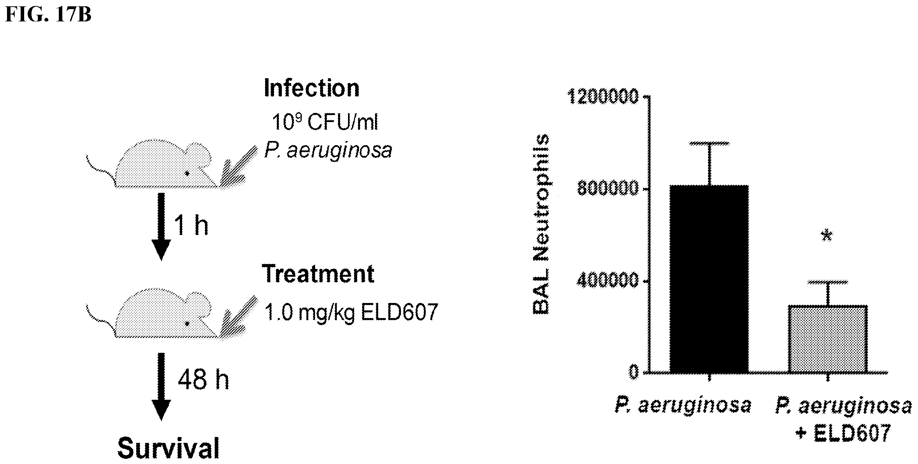

**FIG. 17A** shows a schematic of the infection protocol and a bar graph of % survival of the *P. aeruginosa* infected mice 48 hours post ELD607 treatment. **FIG. 17B** shows a bar graph of BAL neutrophils of the same mice.

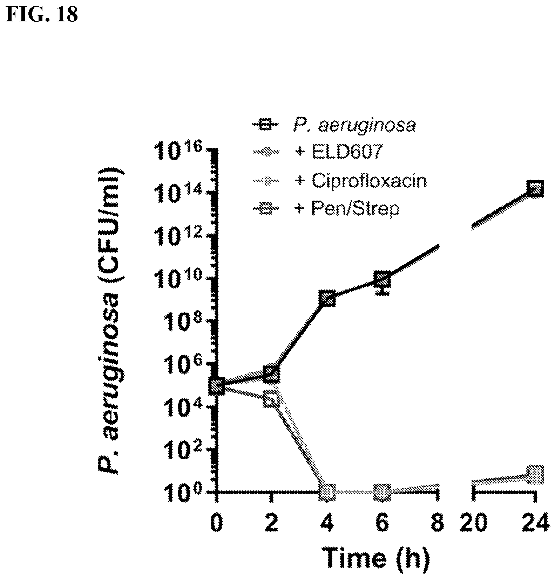

**FIG. 18** shows a graph of *P. aeruginosa* growth in the presence of ELD100 (light circles; reference), ELD607 (triangles), untreated (dark circles; negative control), or penicillin and streptomycin (diamonds; positive control),wherein the antibiotic treatment fully inhibited growth. ELD607 did not demonstrate antimicrobial activity.

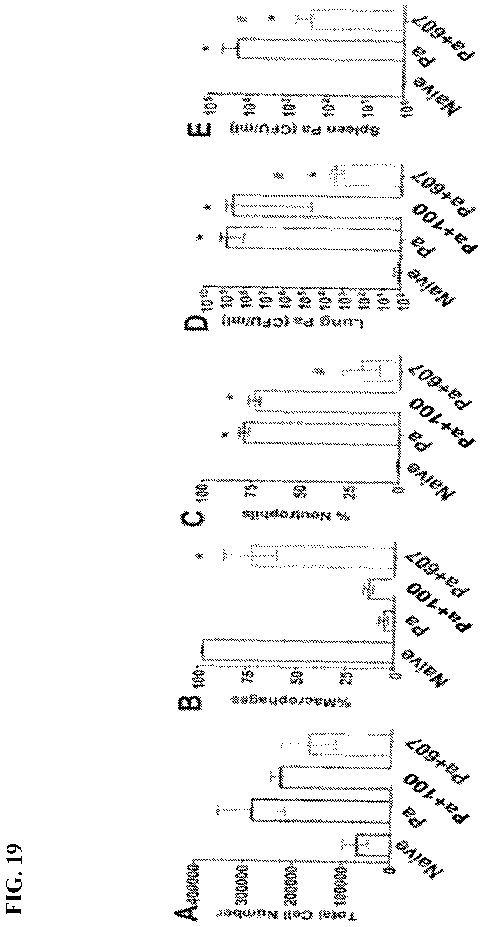

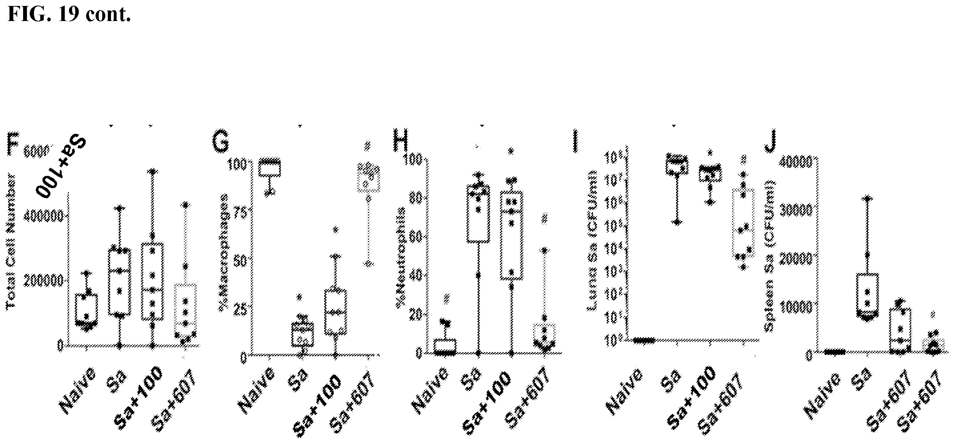

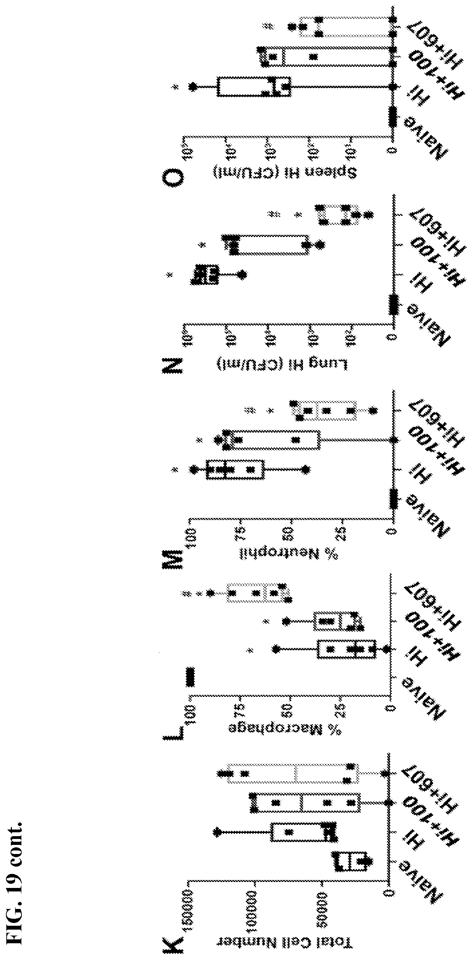

**FIG. 19** shows graphs of BAL, lung and spleen analyses from three different bacterial infections, suggesting that ELD607 broadly reduces neutrophilia and increases bacterial clearance from the lung. Mice were dosed intranasally with *P. aeruginosa (PA), S. aureus (Sa),* or *H. influenzae* followed by vehicle (saline) or 0.5 mg/kg of ELD100 or ELD607 per mouse. **FIG. 19** **panels A, F, and K** show total cell counts; **FIG. 19** **panels B, G, and L** show BAL macrophages; **FIG. 19** **panels C, H, and M** show BAL neutrophils; **FIG. 19** **panels D, I, and N** show whole lung CFUs; and **FIG. 19** **panels E, J, and O** show spleen CFUs. Top row, *P. aeruginosa,* middle row, *S. aureus,* bottom row, *H. influenzae.* * p<0.05 different from naïve mice; # p<0.05 different from infected mice.

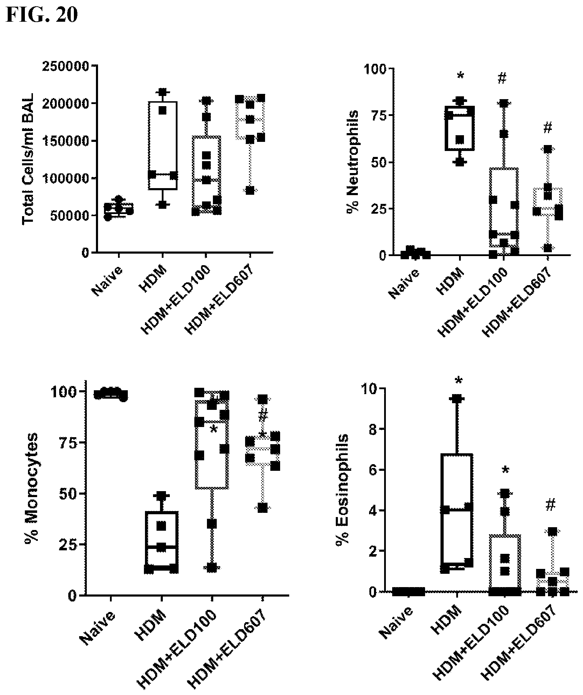

**FIG. 20** shows bar graphs of results of house dust mite (HDM) allergy model experiments, indicating BAL total cell count **(****FIG. 20** **top left panel),** BAL eosinophils **(****FIG. 20** **bottom right panel)** BAL monocytes **(****FIG. 20** **bottom left panel),** and BAL neutrophils **(****FIG. 20** **top right panel)** in HDM-exposed mice treated with or without ELD100 (reference) and ELD607.

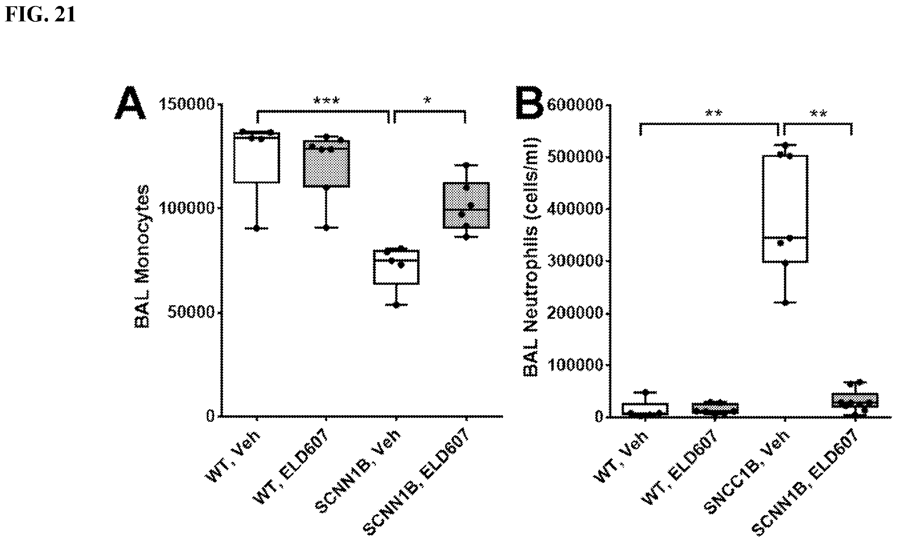

**FIG. 21** shows graphs of BAL macrophages **(****FIG. 21** **panel A)** and neutrophils **(****FIG. 21** **panel B)** in SNCC1B mice, showing that ELD607 reduces pulmonary inflammation in SNCC1B mice. SCNN1B and littermate control mice were exposed to ELD607 or vehicle intranasally on a daily basis for 11 days, starting on day 1 post-partum. ELD607 had no effect on WT mice but significantly decreased BAL neutrophil levels in SCNN1B mice. * p<0.05, *** p<0.001 different as indicated.

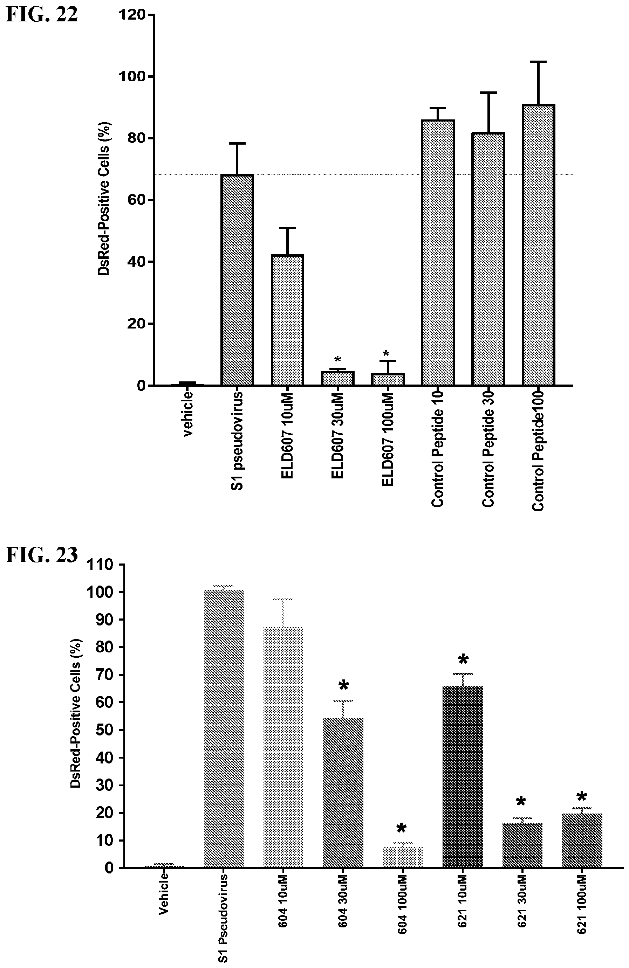

**FIG. 22** shows a bar graph of % infected cells by DsRed-positivity, showing that ELD607 reduces SARS-CoV-2 S1 pseudovirus infection in ACE2-expressing HEK293T cells. All n = 3 per group. * p<0.05 different to S1 pseudovirus infection alone.
**FIG. 23** shows a bar graph of % infected cells by DsRed-positivity. Experiments were performed in the same manner as **FIG. 22****,** with additional peptides noted below each bar. All n = 3 per group. * p<0.05 different to S1 pseudovirus infection alone.

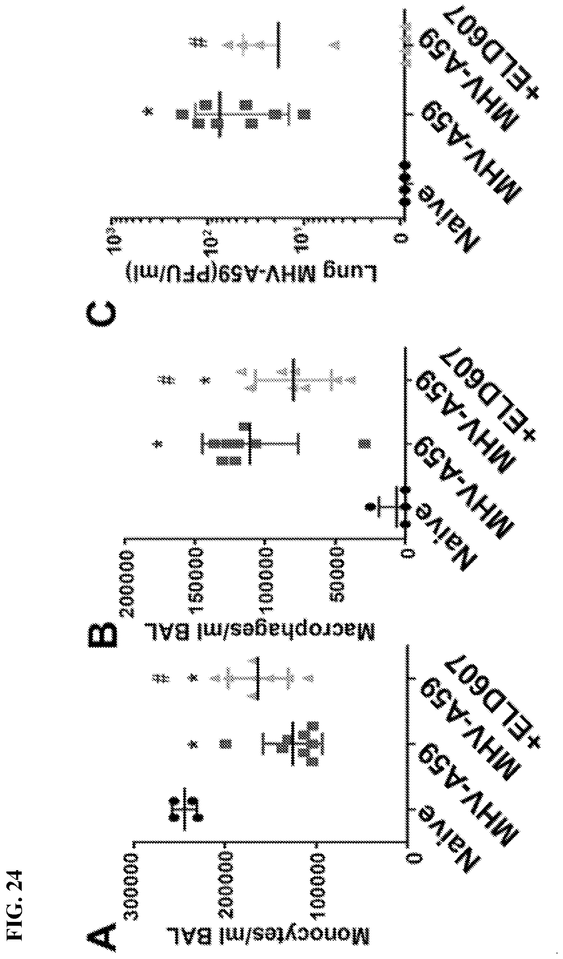

**FIG. 24** shows dot graphs indicating that ELD607 normalizes leukocyte levels and increases viral clearance from the lung. Mice were dosed intranasally with 10⁶ PFU MHV-A59 followed by vehicle (saline) or 1.0 mg/kg of ELD607 per mouse 4 d p.i. BAL and lungs were collected 6 d p.i. **FIG. 24** **panel A** shows BAL monocytes; **FIG. 24** **panel B** shows BAL macrophages; **FIG. 24** **panel C** shows whole lung PFUs. * p<0.05 different from naïve mice; # p<0.05 different from infected mice.

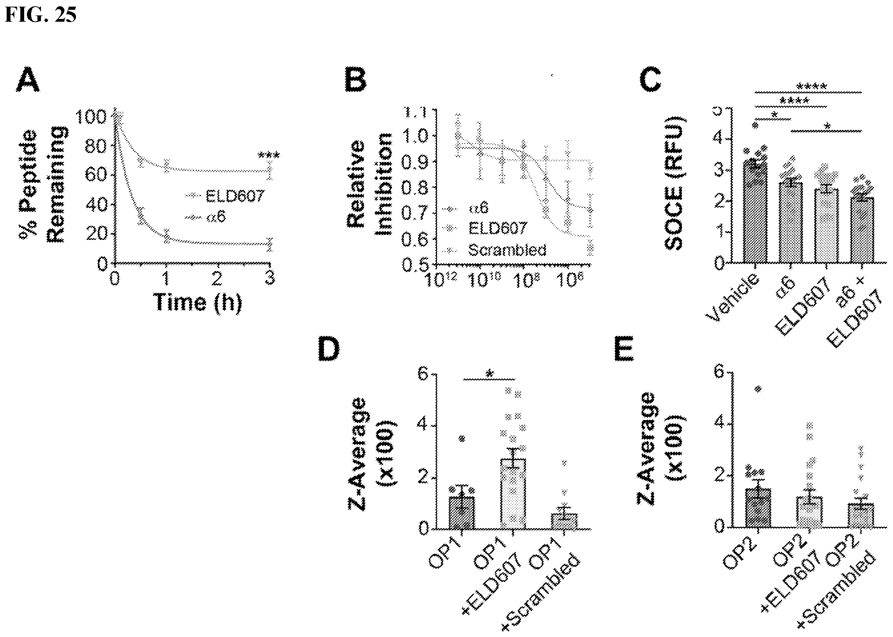

**FIG. 25** shows data plots and bar graphs of ELD607 in HEK293T cell studies. **FIG. 25****, panel A** shows % peptide remaining over time; **FIG. 25****, panel B** shows relative inhibition; **FIG. 25****, panel C** shows store-operated calcium entry (SOCE) by relative fluorescence units (RFU); **FIG. 25****, panel D** shows intensity weight harmonic mean particle size (Z-average) in the presence of OP1; **FIG. 25****, panel E** shows Z-average in the presence of OP2.

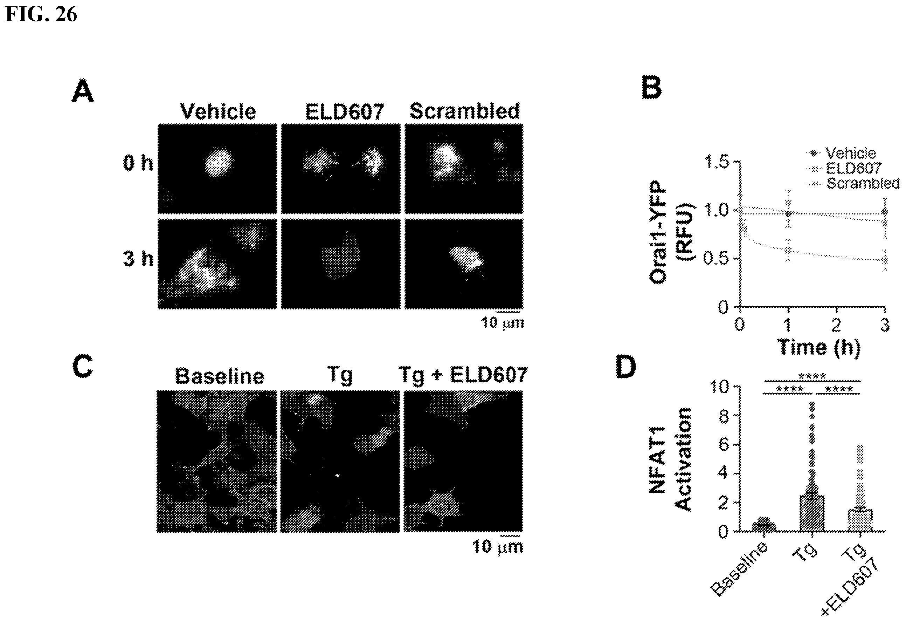

**FIG. 26** shows images of total internal reflection fluorescence (TIFR) microscopy and related data plots regarding Orai1 in the plasma membrane. **FIG. 26** **panel A** shows cell culture images with vehicle, ELD607, and scrambled at 0h and 3h treatment. **FIG. 26** **panel B** shows Orai1-YFP measured by relative fluorescence units, as related to **FIG. 26** **panel A.** **FIG. 26** **panel C** shows cell culture images of baseline, thapsigargin treatment, and thapsigargin + ELD607 treatment. **FIG. 26** **panel D** shows NFAT1 activation levels, as related to **FIG. 26** **panel C.**

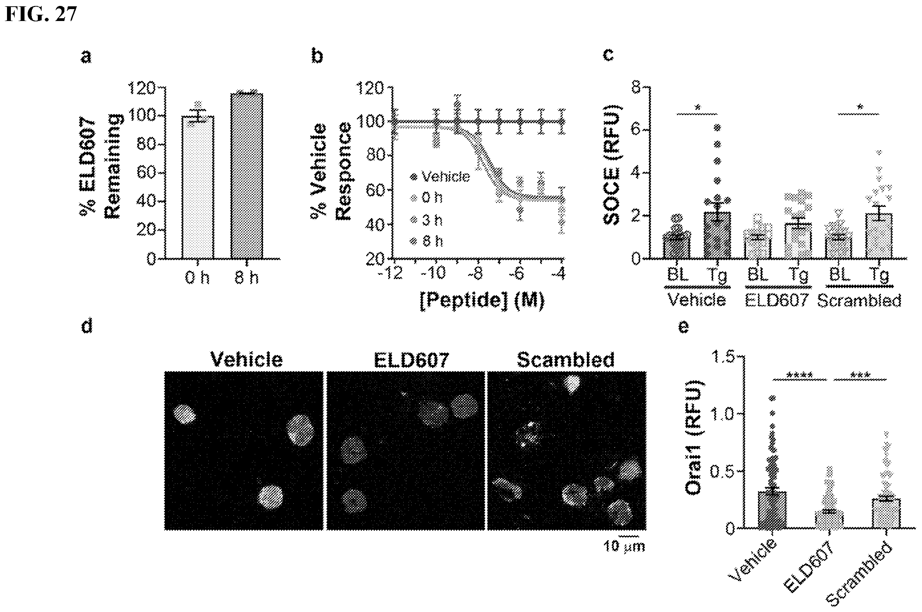

**FIG. 27** shows data plots and fluorescence images regarding ARDS BAL samples. **FIG. 27** **panel A** shows a bar graph of % stable ELD607 remaining after 8 hours at 37 °C. **FIG. 27** **panel B** shows % vehicle response. **FIG. 27** **panel C** shows store-operated calcium entry (SOCE) score per relative fluorescence units. **FIG. 27** **panel D** shows fluorescence images of vehicle, ELD607, and scrambled. **FIG. 27** **panel E** shows Orai1 levels per relative fluorescence units in relation to **FIG. 27** **panel D.**

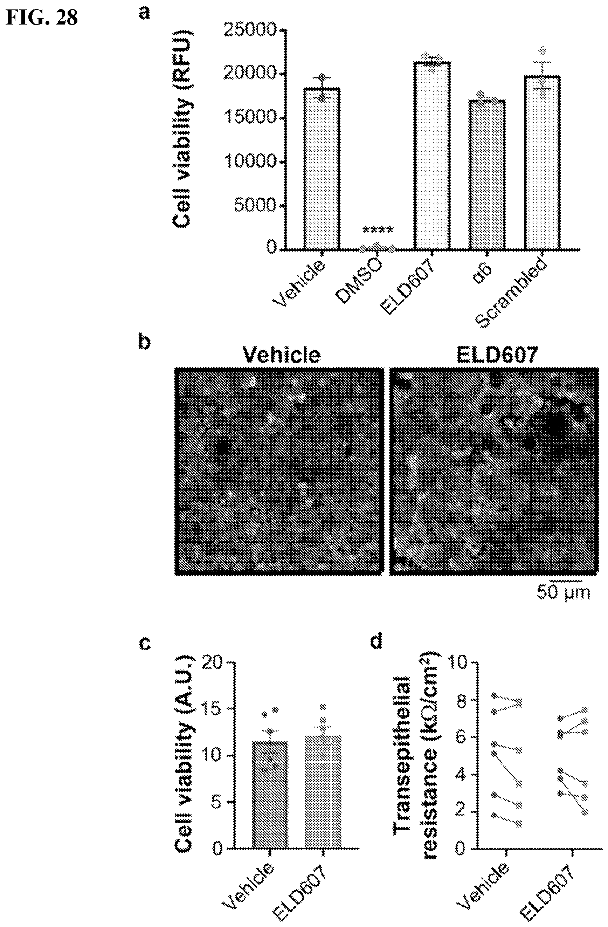

**FIG. 28** shows data plots and fluorescence images regarding experiments in HEK293 T cells exposed to 100 µM ELD607 for 24 hours. **FIG. 28** **panel A** shows a bar graph of cell viability (RFU). **FIG. 28** **panel B** shows fluorescence images of vehicle and ELD607. **FIG. 28** **panels** C and D show cell viability per arbitrary units (left) and transepithelial resistance (right) of vehicle vs. ELD607 before and after treatment.

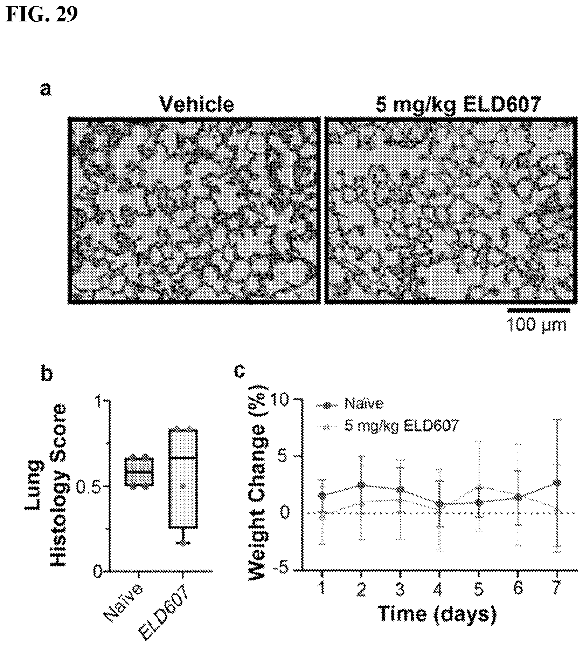

**FIG. 29** shows images of histology and related data plots regarding experiments performed in mice treated intratracheally at 10 mg/kg daily for 7 days. **FIG. 29** **panel A** shows images of the lung. **FIG. 29** **panel B** shows the lung histology score, in relation to **FIG. 29** **panel A.** **FIG. 29** **panel C** shows % weight change of the mice over time in days.

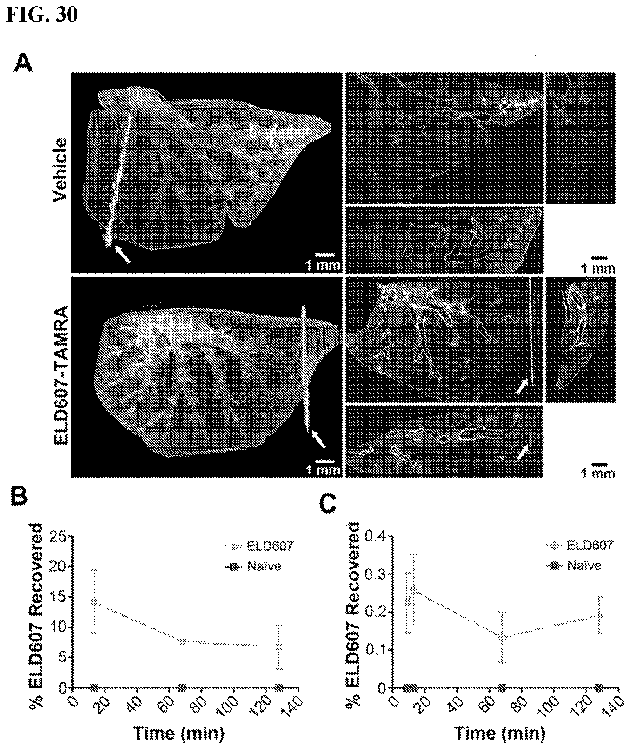

**FIG. 30** shows images of histology and related data plots regarding experiments performed in mice treated intratracheally at 0.5 mM for 1 hour. **FIG. 30** **panel A** shows images of light-sheet fluorescence microscopy in the airway lumen and interstitial. **FIG. 30** **panels B and C** show % ELD607 recovered in BAL **(****FIG. 30** **panel B)** and blood **(****FIG. 30** **panel C).**

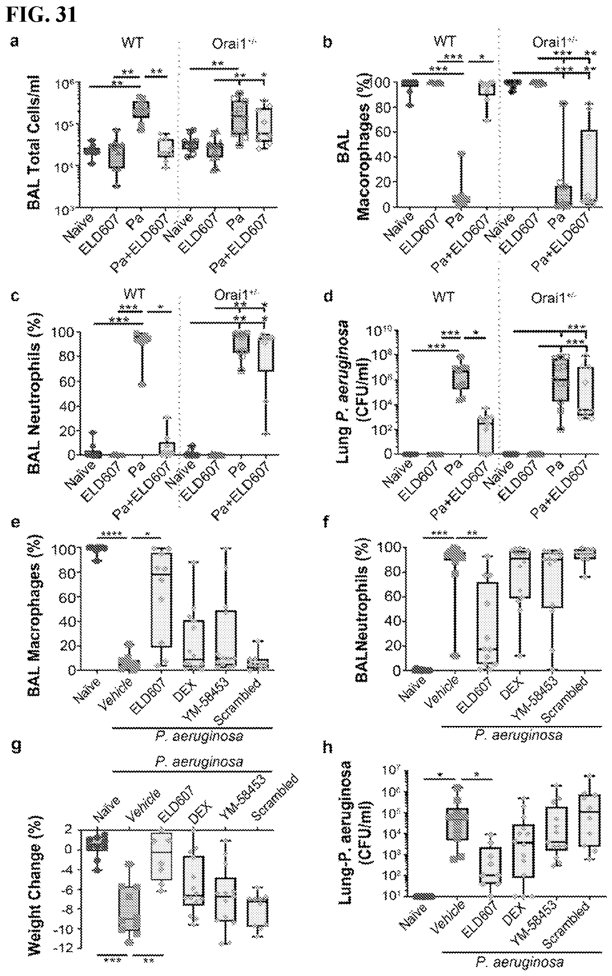

**FIG. 31** shows bar graphs regarding experiments performed in Orai1 heterozygous mice that have about 70% reduced calcium influx, infected with 10⁷ CFU/mouse *P*. *aeruginosa* POA1 and treated intranasally 1 hour later with 0.5 mg/kg ELD607. **FIG. 31** **panel A** shows Bal total cells/ml; **FIG. 31** **panel B** shows BAL macrophages; **FIG. 31** **panel C** shows BAL neutrophils; **FIG. 31** **panel D** shows lung *P. aeruginosa* CFU count/ml; **FIG. 31** **panel E** shows BAL macrophages; **FIG. 31** **panel F** shows BAL neutrophils; **FIG. 31** **panel G** shows % weight change; and **FIG. 31** **panel H** shows lung *P. aeruginosa* CFU count/ml.

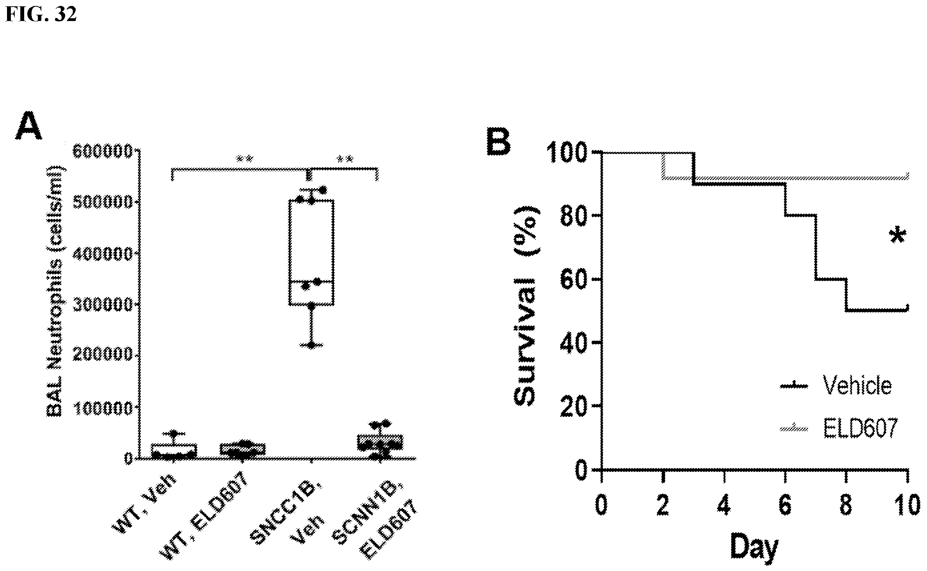

**FIG. 32** shows a data plot and related survival plot regarding ELD607 reduction of BAL neutrophilia when added 24 h after infection and when chronically added to bENaC mice. **FIG. 32** **panel A:** Mice were infected with *P. aeruginosa* for 24 h; then, 0.5 mg/kg ELD607 was added intratracheally. BAL was collected 24 h later to determine cell counts. SCNN1B mice were given 0.5 mg/kg ELD607 daily for 11 days before sacrifice and BAL collection. **FIG. 32** **panel B:** Survival of bENaC mice.

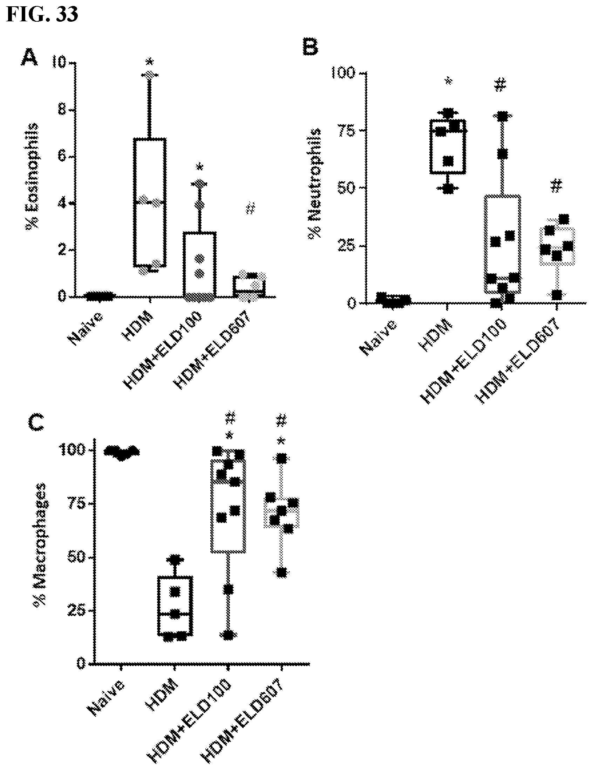

**FIG. 33** shows data plots regarding ELD607 reduction of allergic inflammation in the house dust mite (HDM) model. **FIG. 33** **panel A** shows % eosinophils; **FIG. 33** **panel B** shows % neutrophils; and **FIG. 33** **panel C** shows % macrophages. Mice were exposed to HDM allergen for 14 days, with 3 µg ELD100 (reference) or 2.5 µg ELD607 instilled instranasally for 2 days before sacrifice.

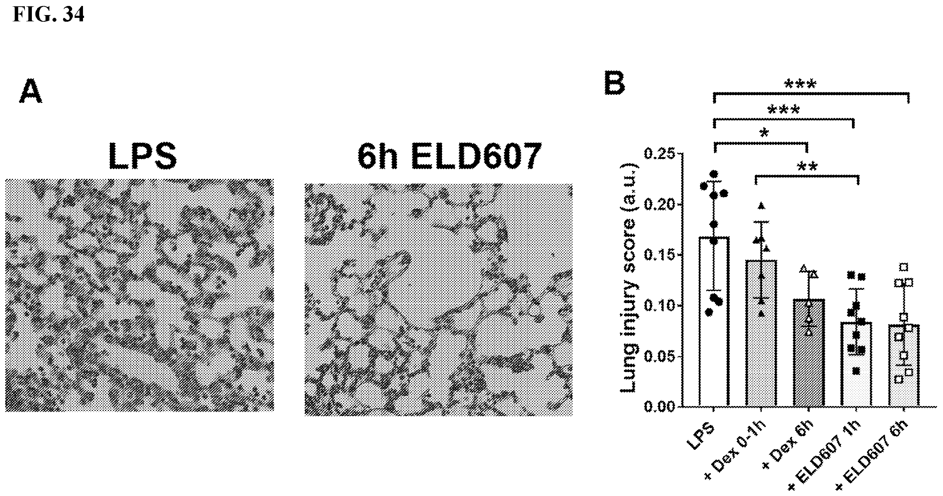

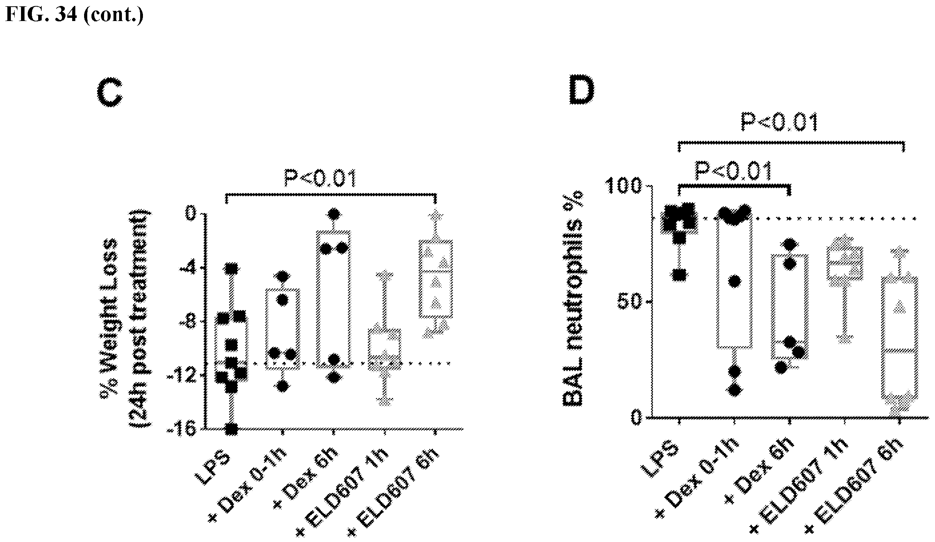

**FIG. 34** shows images of histology and related data plots regarding ELD607 reduction of inflammation in an ARDS model. **FIG. 34** **panel A** shows lung histology after treatment with LPS or 6h of ELD607. **FIG. 34** **panels B-D** show lung injury score **(****FIG. 34** **panel B),** % weight loss **(****FIG. 34** **panel C),** and % BAL neutrophils **(****FIG. 34** **panel C)** in relation to **FIG. 34** **panel A.** Lung injury scoring system taken from Aeffner et al. Mouse Models of Acute Respiratory Distress Syndrome: A Review of Analytical Approaches, Pathologic Features, and Common Measurements. 2015 Tox Pathol.

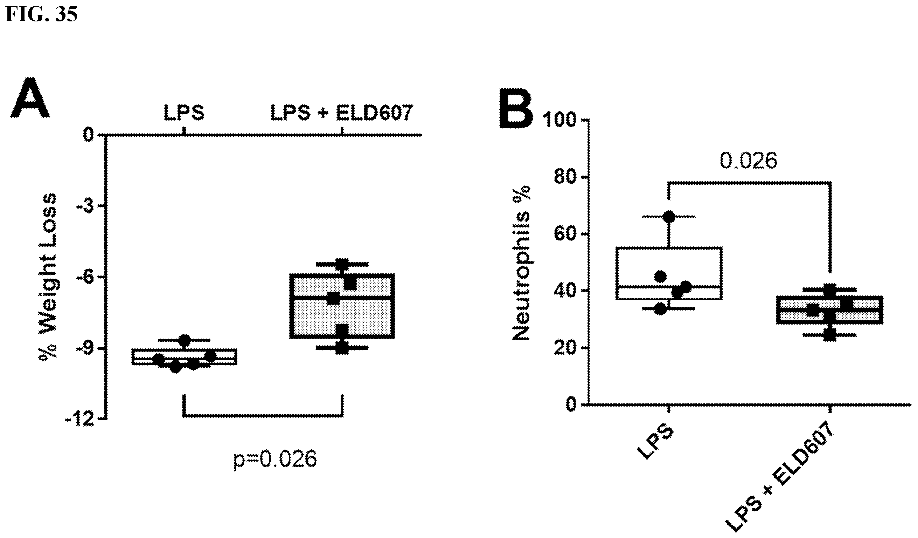

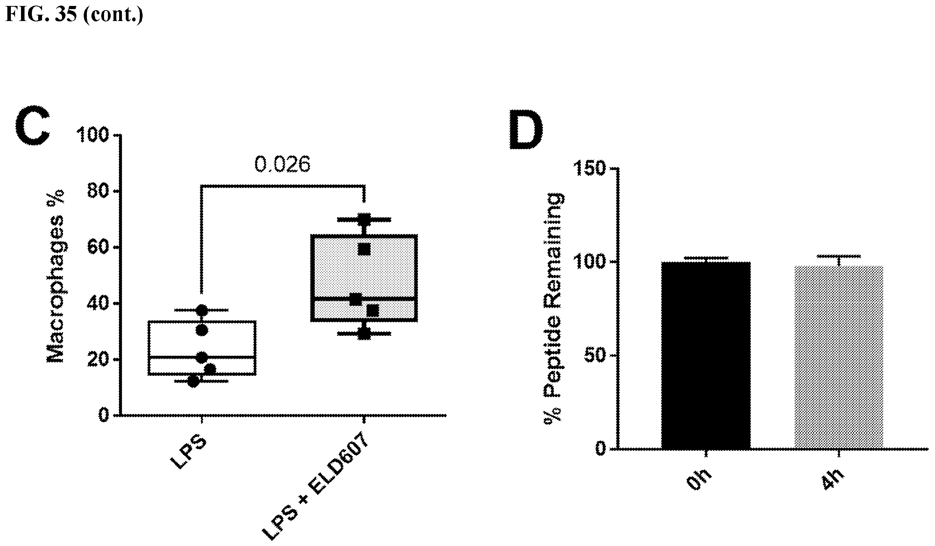

**FIG. 35** shows data plots regarding ELD607 reduction of inflammation in a sepsis model. **FIG. 35** **panel A** shows % weight loss, **FIG. 35** **panel B** shows % neutrophils, **FIG. 35** **panel C** shows % macrophages, and **FIG. 35** **panel D** shows % peptide remaining in experiments wherein mice were intraperitoneally injected with *E. coli* LPS, followed by 0.5 mg/kg ELD607 injected intravenously 1 h later.

### DETAILED DESCRIPTION

The present invention now will be described hereinafter with reference to the accompanying drawings and examples, in which embodiments of the invention are shown. This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. Thus, the invention contemplates that in some embodiments of the invention, any feature or combination of features set forth herein can be excluded or omitted. In addition, numerous variations and additions to the various embodiments suggested herein will be apparent to those skilled in the art in light of the instant disclosure, which do not depart from the instant invention. Hence, the following descriptions are intended to illustrate some particular embodiments of the invention, and not to exhaustively specify all permutations, combinations, and variations thereof.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

Unless the context indicates otherwise, it is specifically intended that the various features of the invention described herein can be used in any combination. Moreover, the present invention also contemplates that in some embodiments of the invention, any feature or combination of features set forth herein can be excluded or omitted. To illustrate, if the specification states that a composition comprises components A, B and C, it is specifically intended that any of A, B or C, or a combination thereof, can be omitted and disclaimed singularly or in any combination.

As used in the description of the invention and the appended claims, the singular forms "a," "an" and "the" are intended to include the plural forms as well, unless the context clearly indicates otherwise.

Also as used herein, "and/or" refers to and encompasses any and all possible combinations of one or more of the associated listed items, as well as the lack of combinations when interpreted in the alternative ("or").

The term "about," as used herein when referring to a measurable value such as an amount or concentration and the like, is meant to encompass variations of ± 10%, ± 5%, ± 1%, ± 0.5%, or even ± 0.1% of the specified value as well as the specified value. For example, "about X" where X is the measurable value, is meant to include X as well as variations of ± 10%, ± 5%, ± 1%, ± 0.5%, or even ± 0.1% of X. A range provided herein for a measurable value may include any other range and/or individual value therein.

As used herein, phrases such as "between X and Y" and "between about X and Y" should be interpreted to include X and Y. As used herein, phrases such as "between about X and Y" mean "between about X and about Y" and phrases such as "from about X to Y" mean "from about X to about Y."

The term "comprise," "comprises" and "comprising" as used herein, specify the presence of the stated features, integers, steps, operations, elements, and/or components, but do not preclude the presence or addition of one or more other features, integers, steps, operations, elements, components, and/or groups thereof.

As used herein, the transitional phrase "consisting essentially of" means that the scope of a claim is to be interpreted to encompass the specified materials or steps recited in the claim and those that do not materially affect the basic and novel characteristic(s) of the claimed invention. Thus, the term "consisting essentially of" when used in a claim of this invention is not intended to be interpreted to be equivalent to "comprising."

The term "consists essentially of" (and grammatical variants), as applied to a peptide sequence of this invention, means a peptide that consists of both the recited sequence (e.g., SEQ ID NO) and a total of ten or less *(e.g.,* 1, 2, 3, 4, 5, 6, 7, 8, 9, or 10) additional amino acids on the N-terminal and/or C-terminal ends of the recited sequence such that the function of the peptide is not materially altered. The total of ten or less additional amino acids includes the total number of additional amino acids on both ends added together. The term "materially altered," as applied to peptides of the invention, refers to an increase or decrease in binding activity *(e.g.,* to a calcium channel) of at least about 50% or more as compared to the activity of a peptide consisting of the recited sequence.

Amino acids are represented herein in the manner recommended by the IUPAC-IUB Biochemical Nomenclature Commission, or (for amino acids) by either the one-letter code, or the three letter code, both in accordance with 37 C.F.R. §1.822 and established usage, e.g., as shown in Table 6.

The term "modulate," "modulates," or "modulation" refers to enhancement (*e.g*., an increase) or inhibition (*e.g.*, a decrease) in the specified level or activity.

The term "enhance" or "increase" refers to an increase in the specified parameter of at least about 1.25-fold, 1.5-fold, 2-fold, 3-fold, 4-fold, 5-fold, 6-fold, 8-fold, 10-fold, twelve-fold, or even fifteen-fold.

The term "inhibit" or "reduce" or grammatical variations thereof as used herein refers to a decrease or diminishment in the specified level or activity of at least about 15%, 25%, 35%, 40%, 50%, 60%, 75%, 80%, 90%, 95% or more. In particular embodiments, the inhibition or reduction results in little or essentially no detectible activity (at most, an insignificant amount, *e.g.,* less than about 10% or even 5%).

The term "contact" or grammatical variations thereof as used with respect to a polypeptide and a calcium channel, refers to bringing the polypeptide and the calcium channel in sufficiently close proximity to each other for one to exert a biological effect on the other. In some embodiments, the term contact means binding of the polypeptide to the calcium channel.

By the terms "treat," "treating," or "treatment of," it is intended that the severity of the subject's condition is reduced or at least partially improved or modified and that some alleviation, mitigation or decrease in at least one clinical symptom is achieved.

The terms "prevent," "preventing," and "prevention" (and grammatical variations thereof) refer to prevention and/or delay of the onset of a disease, disorder and/or a clinical symptom(s) in a subject and/or a reduction in the severity of the onset of the disease, disorder and/or clinical symptom(s) relative to what would occur in the absence of the methods of the invention. The prevention can be complete, *e.g.,* the total absence of the disease, disorder and/or clinical symptom(s). The prevention can also be partial, such that the occurrence of the disease, disorder and/or clinical symptom(s) in the subject and/or the severity of onset is less than what would occur in the absence of the present invention.

A "therapeutically effective" or "effective" amount as used herein is an amount that provides some improvement or benefit to the subject. Alternatively stated, a "therapeutically effective" amount or an "effective amount" is an amount that will provide some alleviation, mitigation, or decrease in at least one clinical symptom in the subject. Those skilled in the art will appreciate that the effects need not be complete or curative, as long as some benefit is provided to the subject.

A "prophylactically effective" amount as used herein is an amount that is sufficient to prevent and/or delay the onset of a disease, disorder and/or clinical symptoms in a subject and/or to reduce and/or delay the severity of the onset of a disease, disorder and/or clinical symptoms in a subject relative to what would occur in the absence of the methods of the invention. Those skilled in the art will appreciate that the level of prevention need not be complete, as long as some benefit is provided to the subject.

The term "fragment," as applied to a peptide, will be understood to mean an amino acid sequence of reduced length relative to a reference peptide or amino acid sequence and comprising, consisting essentially of, and/or consisting of an amino acid sequence of contiguous amino acids identical to the reference peptide or amino acid sequence. Such a peptide fragment according to the invention may be, where appropriate, included in a larger polypeptide of which it is a constituent. In some embodiments, such fragments can comprise, consist essentially of, and/or consist of peptides having a length of at least about 4, 5, 6, 7, 8, 9, 10, 11, 12, 13, 14, 15 or more consecutive amino acids of a peptide or amino acid sequence according to the invention. In other embodiments, such fragments can comprise, consist essentially of, and/or consist of peptides having a length of less than about 15, 14, 13, 12, 11, 10, 9, 8, 7, 6, 5, 4, or less consecutive amino acids of a peptide or amino acid sequence according to the invention.

As used herein, the terms "protein" and "polypeptide" are used interchangeably and encompass both peptides and proteins, unless indicated otherwise.

The "N-terminus" of a polypeptide is any portion of the polypeptide that starts from the N-terminal amino acid residue and continues to a maximum of the midpoint of the polypeptide.

The "C-terminus" of a polypeptide is any portion of the polypeptide that starts from the C-terminal amino acid residue and continues to a maximum of the midpoint of the polypeptide.

As used herein, a "functional" peptide or "functional fragment" is one that substantially retains at least one biological activity normally associated with that peptide (e.g., binding to or inhibiting a calcium channel). In particular embodiments, the "functional" peptide or "functional fragment" substantially retains all of the activities possessed by the unmodified peptide. By "substantially retains" biological activity, it is meant that the peptide retains at least about 50%, 60%, 75%, 85%, 90%, 95%, 97%, 98%, 99%, or more, of the biological activity of the native polypeptide (and can even have a higher level of activity than the native peptide). A "non-functional" peptide is one that exhibits little or essentially no detectable biological activity normally associated with the peptide *(e.g.,* at most, only an insignificant amount, *e.g.,* less than about 10% or even 5%). Biological activities such as protein binding and calcium channel inhibitory activity can be measured using assays that are well known in the art and as described herein.

A "fusion protein" is a polypeptide produced when two heterologous nucleotide sequences or fragments thereof coding for two (or more) different polypeptides not found fused together in nature are fused together in the correct translational reading frame. Illustrative fusion polypeptides include fusions of a peptide of the invention (or a fragment thereof) to all or a portion of glutathione-S-transferase, maltose-binding protein, or a reporter protein *(e.g.,* Green Fluorescent Protein, β-glucuronidase, β-galactosidase, luciferase, *etc.),* hemagglutinin, c-myc, FLAG epitope, *etc.*

As used herein, "nucleic acid molecule," "nucleotide sequence," and "polynucleotide" are used interchangeably and encompass both RNA and DNA, including cDNA, genomic DNA, mRNA, synthetic (e.g., chemically synthesized) DNA or RNA and chimeras of RNA and DNA. The term nucleic acid refers to a chain of nucleotides without regard to length of the chain. The nucleic acid can be double-stranded or single-stranded. Where single-stranded, the nucleic acid can be a sense strand or an antisense strand. The nucleic acid can be synthesized using oligonucleotide analogs or derivatives (e.g., inosine or phosphorothioate nucleotides). Such oligonucleotides can be used, for example, to prepare nucleic acids that have altered base-pairing abilities or increased resistance to nucleases. The present invention further provides a nucleic acid that is the complement (which can be either a full complement or a partial complement) of a nucleic acid or nucleotide sequence of this invention.

An "isolated polynucleotide" is a nucleotide sequence (e.g., DNA or RNA) that is not immediately contiguous with nucleotide sequences with which it is immediately contiguous (one on the 5' end and one on the 3' end) in the naturally occurring genome of the organism from which it is derived. Thus, in one embodiment, an isolated nucleic acid includes some or all of the 5' non-coding (e.g., promoter) sequences that are immediately contiguous to a coding sequence. The term therefore includes, for example, a recombinant DNA that is incorporated into a vector, into an autonomously replicating plasmid or virus, or into the genomic DNA of a prokaryote or eukaryote, or which exists as a separate molecule *(e.g.,* a cDNA or a genomic DNA fragment produced by PCR or restriction endonuclease treatment), independent of other sequences. It also includes a recombinant DNA that is part of a hybrid nucleic acid encoding an additional polypeptide or peptide sequence. An isolated polynucleotide that includes a gene is not a fragment of a chromosome that includes such gene, but rather includes the coding region and regulatory regions associated with the gene, but no additional genes naturally found on the chromosome.

The term "isolated" can refer to a nucleic acid, nucleotide sequence or polypeptide that is substantially free of cellular material, viral material, and/or culture medium (when produced by recombinant DNA techniques), or chemical precursors or other chemicals (when chemically synthesized). Moreover, an "isolated fragment" is a fragment of a nucleic acid, nucleotide sequence or polypeptide that is not naturally occurring as a fragment and would not be found in the natural state. "Isolated" does not mean that the preparation is technically pure (homogeneous), but it is sufficiently pure to provide the polypeptide or nucleic acid in a form in which it can be used for the intended purpose.

An isolated cell refers to a cell that is separated from other components with which it is normally associated in its natural state. For example, an isolated cell can be a cell in culture medium and/or a cell in a pharmaceutically acceptable carrier of this invention. Thus, an isolated cell can be delivered to and/or introduced into a subject. In some embodiments, an isolated cell can be a cell that is removed from a subject and manipulated as described herein *ex vivo* and then returned to the subject.

A "vector" is any nucleic acid molecule for the cloning of and/or transfer of a nucleic acid into a cell. A vector may be a replicon to which another nucleotide sequence may be attached to allow for replication of the attached nucleotide sequence. A "replicon" can be any genetic element (e.g., plasmid, phage, cosmid, chromosome, viral genome) that functions as an autonomous unit of nucleic acid replication *in vivo, i.e.,* capable of replication under its own control. The term "vector" includes both viral and nonviral (e.g., plasmid) nucleic acid molecules for introducing a nucleic acid into a cell *in vitro, ex vivo,* and/or *in vivo.* A large number of vectors known in the art may be used to manipulate nucleic acids, incorporate response elements and promoters into genes, *etc.* For example, the insertion of the nucleic acid fragments corresponding to response elements and promoters into a suitable vector can be accomplished by ligating the appropriate nucleic acid fragments into a chosen vector that has complementary cohesive termini. Alternatively, the ends of the nucleic acid molecules may be enzymatically modified or any site may be produced by ligating nucleotide sequences (linkers) to the nucleic acid termini. Such vectors may be engineered to contain sequences encoding selectable markers that provide for the selection of cells that contain the vector and/or have incorporated the nucleic acid of the vector into the cellular genome. Such markers allow identification and/or selection of host cells that incorporate and express the proteins encoded by the marker. A "recombinant" vector refers to a viral or non-viral vector that comprises one or more heterologous nucleotide sequences *(i.e.,* transgenes), *e.g.,* two, three, four, five or more heterologous nucleotide sequences.

Viral vectors have been used in a wide variety of gene delivery applications in cells, as well as living animal subjects. Viral vectors that can be used include, but are not limited to, retrovirus, lentivirus, adeno-associated virus, poxvirus, alphavirus, baculovirus, vaccinia virus, herpes virus, Epstein-Barr virus, and adenovirus vectors. Non-viral vectors include plasmids, liposomes, electrically charged lipids (cytofectins), nucleic acid-protein complexes, and biopolymers. In addition to a nucleic acid of interest, a vector may also comprise one or more regulatory regions, and/or selectable markers useful in selecting, measuring, and monitoring nucleic acid transfer results (delivery to specific tissues, duration of expression, *etc.).*

Vectors may be introduced into the desired cells by methods known in the art, *e.g.,* transfection, electroporation, microinjection, transduction, cell fusion, DEAE dextran, calcium phosphate precipitation, lipofection (lysosome fusion), use of a gene gun, or a nucleic acid vector transporter *(see, e.g.,* Wu et al., J. Biol. Chem. 267:963 (1992); Wu et al., J. Biol. Chem. 263:14621 (1988); and Hartmut et al., Canadian Patent Application No. 2,012,311, filed Mar. 15, 1990). In various embodiments, other molecules can be used for facilitating delivery of a nucleic acid *in vivo,* such as a cationic oligopeptide *(e.g.,* WO95/21931), peptides derived from nucleic acid binding proteins (e.g., WO96/25508), and/or a cationic polymer *(e.g.,* WO95/21931). It is also possible to introduce a vector *in vivo* as naked nucleic acid (see U.S. Patent Nos. 5,693,622, 5,589,466 and 5,580,859). Receptor-mediated nucleic acid delivery approaches can also be used (Curiel et al., Hum. Gene Ther. 3:147 (1992); Wu et al., J. Biol. Chem. 262:4429 (1987)).

The term "transfection" or "transduction" means the uptake of exogenous or heterologous nucleic acid (RNA and/or DNA) by a cell. A cell has been "transfected" or "transduced" with an exogenous or heterologous nucleic acid when such nucleic acid has been introduced or delivered inside the cell. A cell has been "transformed" by exogenous or heterologous nucleic acid when the transfected or transduced nucleic acid imparts a phenotypic change in the cell and/or a change in an activity or function of the cell. The transforming nucleic acid can be integrated (covalently linked) into chromosomal DNA making up the genome of the cell or it can be present as a stable plasmid.

By the term "express" or "expression" of a polynucleotide coding sequence, it is meant that the sequence is transcribed, and optionally, translated. Typically, according to the present invention, expression of a coding sequence of the invention will result in production of the polypeptide of the invention. The entire expressed polypeptide can also function in intact cells without purification.

### Compositions

The present invention relates to the design of peptidomimetics that bind to and inhibit the Orai1 calcium channel. Orai1 is a Ca²⁺ channel and master regulator of inflammation upstream of cytokine responses **(****FIG. 1****).** Therefore, selective inhibition of Orai1 can be immunomodulatory.

Global Orai1 expression presents a problem of potential off-target effects when drug-targeted. Orai1 regulates Ca²⁺ homeostasis in immune cells, where activation of the endoplasmic reticulum (ER) calcium sensor stromal interaction molecule 1 (STIM1) via ER calcium depletion downstream of T Cell Receptor (TCR) stimulation leads to Orai1-dependent calcium transport into the cell, which in turn activates calcineurin (a calcium and calmodulin dependent serine/threonine protein phosphatase)-dependent immune responses such as interleukin (IL)-8, IL-2, and tumor necrosis factor-alpha (TNF-α) **(****FIG. 2****).**

While not wishing to be bound to theory, it is believed that the synthetic polypeptides of the present invention such as ELD607 may inhibit Orai1 and thereby attenuate Ca²⁺-dependent inflammation. ELD607 may also modulate immune responses against viruses which stimulate immune responses which depend on calcium fluxes. For example, COVID-19, the infection caused by SARS-CoV-2, causes damage to the infected subject via cytokine storm, pulmonary infiltrates, neutrophilia, and/or lung injury such as acute respiratory distress syndrome (ARDS). Treatment with synthetic polypeptides of the present invention, therefore, may reduce the levels of lung cytokines, decrease neutrophilia, and/or reduce lung damage, resulting in an improved infectious outcome **(****FIG. 3****).**

A synthetic polypeptide of the present invention is a synthetic polypeptide consisting of: the amino acid sequence VHDIVNMLIRG **(SEQ ID NO:8);** or the C-terminally amidated form of amino acid sequence VHDIVNMLIRG **(SEQ ID NO:8).**
**SEQ ID NO:8. ELD607.**
VHDIVNMLIRG

The present invention further provides an isolated nucleic acid molecule encoding the synthetic polypeptide of this invention. In some embodiments, a nucleic acid molecule of this invention may be a cDNA molecule. In some embodiments, a nucleic acid molecule of this invention may be an mRNA molecule. It will be appreciated by those skilled in the art that there can be variability in the nucleic acid molecules that encode the polypeptides (and fragments thereof) of the present invention due to the degeneracy of the genetic code. The degeneracy of the genetic code, which allows different nucleic acid sequences to code for the same polypeptide, is well known in the literature *(See, e.g.,* **Table 6).**

As is known in the art, a number of different programs can be used to identify whether a nucleic acid molecule or polypeptide has sequence identity or similarity to a known sequence. Sequence identity or similarity may be determined using standard techniques known in the art, including, but not limited to, the local sequence identity algorithm of Smith & Waterman, Adv. Appl. Math. 2:482 (1981), by the sequence identity alignment algorithm of Needleman & Wunsch, J. Mol. Biol. 48:443 (1970), by the search for similarity method of Pearson & Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12:387 (1984), preferably using the default settings, or by inspection.

An example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, J. Mol. Evol. 35:351 (1987); the method is similar to that described by Higgins & Sharp, CABIOS 5:151 (1989).

Another example of a useful algorithm is the BLAST algorithm, described in Altschul et al., J. Mol. Biol. 215:403 (1990) and Karlin et al., Proc. Natl. Acad. Sci. USA 90:5873 (1993). A particularly useful BLAST program is the WU-BLAST-2 program which was obtained from Altschul et al., Meth. Enzymol., 266:460 (1996); blast.wustl/edu/blast/README.html. WU-BLAST-2 uses several search parameters, which are preferably set to the default values. The parameters are dynamic values and are established by the program itself depending upon the composition of the particular sequence and composition of the particular database against which the sequence of interest is being searched; however, the values may be adjusted to increase sensitivity. An additional useful algorithm is gapped BLAST as reported by Altschul et al., Nucleic Acids Res. 25:3389 (1997).

A percentage amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

In a similar manner, percent nucleic acid sequence identity with respect to the coding sequence of the polypeptides disclosed herein is defined as the percentage of nucleotide residues in the candidate sequence that are identical with the nucleotides in the polynucleotide specifically disclosed herein.

The alignment may include the introduction of gaps in the sequences to be aligned. In addition, for sequences which contain either more or fewer amino acids than the polypeptides specifically disclosed herein, it is understood that in one embodiment, the percentage of sequence identity will be determined based on the number of identical amino acids in relation to the total number of amino acids. Thus, for example, sequence identity of sequences shorter than a sequence specifically disclosed herein, will be determined using the number of amino acids in the shorter sequence, in one embodiment. In percent identity calculations relative weight is not assigned to various manifestations of sequence variation, such as insertions, deletions, substitutions, *etc.*

In one embodiment, only identities are scored positively (+1) and all forms of sequence variation including gaps are assigned a value of "0," which obviates the need for a weighted scale or parameters as described below for sequence similarity calculations. Percent sequence identity can be calculated, for example, by dividing the number of matching identical residues by the total number of residues of the "shorter" sequence in the aligned region and multiplying by 100. The "longer" sequence is the one having the most actual residues in the aligned region.

Those skilled in the art will appreciate that the isolated nucleic acid molecules encoding the polypeptides of the invention will typically be associated with appropriate expression control sequences, *e.g.*, transcription/translation control signals and polyadenylation signals.

It will further be appreciated that a variety of promoter/enhancer elements can be used depending on the level and tissue-specific expression desired. The promoter can be constitutive or inducible, depending on the pattern of expression desired. The promoter can be native or foreign and can be a natural or a synthetic sequence. By foreign, it is intended that the transcriptional initiation region is not found in the wild-type host into which the transcriptional initiation region is introduced. The promoter is chosen so that it will function in the target cell(s) of interest.

To illustrate, the polypeptide coding sequence can be operatively associated with a cytomegalovirus (CMV) major immediate-early promoter, an albumin promoter, an Elongation Factor 1-α (EF1-α) promoter, a PyK promoter, a MFG promoter, or a Rous sarcoma virus promoter.

Inducible promoter/enhancer elements include hormone-inducible and metal-inducible elements, and other promoters regulated by exogenously supplied compounds, including without limitation, the zinc-inducible metallothionein (MT) promoter; the dexamethasone (Dex)-inducible mouse mammary tumor virus (MMTV) promoter; the T7 polymerase promoter system *(see* WO 98/10088); the ecdysone insect promoter (No et al., Proc. Natl. Acad. Sci. USA 93:3346 (1996)); the tetracycline-repressible system (Gossen et al., Proc. Natl. Acad. Sci. USA 89:5547 (1992)); the tetracycline-inducible system (Gossen et al., Science 268:1766 (1995); *see also* Harvey et al., Curr. Opin. Chem. Biol. 2:512 (1998)); the RU486-inducible system (Wang et al., Nat. Biotech. 15:239 (1997); Wang et al., Gene Ther., 4:432 (1997)); and the rapamycin-inducible system (Magari et al., J. Clin. Invest. 100:2865 (1997)).

Moreover, specific initiation signals are generally required for efficient translation of inserted polypeptide coding sequences. These translational control sequences, which can include the ATG initiation codon and adjacent sequences, can be of a variety of origins, both natural and synthetic.

The present invention further provides cells comprising the isolated nucleic acid molecules and/or synthetic polypeptides of the invention, or a vector comprising the same. The cell may be a cultured cell or a cell *in vivo, e.g.,* for use in therapeutic methods, diagnostic methods, screening methods, methods for studying the biological action of the Orai1 calcium channel, in methods of producing the synthetic polypeptides, or in methods of maintaining or amplifying the nucleic acid molecules of the invention, *etc.* In another embodiment, the cell is an *ex vivo* cell that has been isolated from a subject. The *ex vivo* cell may be modified and then reintroduced into the subject for diagnostic or therapeutic purposes. In some embodiments, the cell may be an untransformed airway smooth muscle cell or a cell from an airway smooth muscle cell line.

Human subjects include neonates, infants, juveniles, and adults. Optionally, the subject is "in need of" the methods of the present invention, *e.g*., because the subject has or is believed at risk for a disorder including those described herein or that would benefit from the delivery of a synthetic polypeptide including those described herein. As a further option, the subject can be a laboratory animal and/or an animal model of disease.

Also provided is a vector, plasmid or other nucleic acid construct comprising the isolated nucleic acid molecule of this invention.

A vector can be any suitable means for delivering a polynucleotide to a cell. A vector of this invention can be an expression vector that contains all of the genetic components required for expression of the nucleic acid in cells into which the vector has been introduced, as are well known in the art. For example, an expression vector may typically contain an "expression cassette," which includes, in the 5' to 3' direction, a promoter, a coding sequence encoding a polypeptide operatively associated with the promoter, and, optionally, a termination sequence including a stop signal for RNA polymerase and a polyadenylation signal for polyadenylase.

Non-limiting examples of promoters of this invention include CYC1, HIS3, GAL1, GAL4, GAL10, ADH1, PGK, PHO5, GAPDH, ADC1, TRP1, URA3, LEU2, ENO, TPI, and alkaline phosphatase promoters (useful for expression in *Saccharomyces);* AOX1 promoter (useful for expression in *Pichia);* β-lactamase, lac, ara, tet, trp, IP_{L}, IP_{R}, T7, tac, and trc promoters (useful for expression in *Escherichia coli);* light regulated-, seed specific-, pollen specific-, ovary specific-, pathogenesis or disease related-promoters, cauliflower mosaic virus 35S, CMV 35S minimal, cassaya vein mosaic virus (CsVMV), chlorophyll a/b binding protein, ribulose 1,5-bisphosphate carboxylase, shoot-specific promoters, root specific promoters, chitinase, stress inducible promoters, rice tungro bacilliform virus, plant super-promoter, potato leucine aminopeptidase, nitrate reductase, mannopine synthase, nopaline synthase, ubiquitin, zein protein, and anthocyanin promoters (useful for expression in plant cells). Further examples of animal and mammalian promoters known in the art include, but are not limited to, the SV40 early (SV40e) promoter region, the promoter contained in the 3' long terminal repeat (LTR) of Rous sarcoma virus (RSV), the promoters of the E1A or major late promoter (MLP) genes of adenoviruses (Ad), the cytomegalovirus (CMV) early promoter, the herpes simplex virus (HSV) thymidine kinase (TK) promoter, baculovirus IE1 promoter, elongation factor 1 alpha (EF1) promoter, phosphoglycerate kinase (PGK) promoter, ubiquitin (Ubc) promoter, an albumin promoter, the regulatory sequences of the mouse metallothionein-L promoter and transcriptional control regions, the ubiquitous promoters (HPRT, vimentin, α-actin, tubulin and the like), the promoters of the intermediate filaments (desmin, neurofilaments, keratin, GFAP, and the like), the promoters of therapeutic genes (of the MDR, CFTR or factor VIII type, and the like), pathogenesis and/or disease-related promoters, and promoters that exhibit tissue specificity, such as the elastase I gene control region, which is active in pancreatic acinar cells; the insulin gene control region active in pancreatic beta cells, the immunoglobulin gene control region active in lymphoid cells, the mouse mammary tumor virus control region active in testicular, breast, lymphoid and mast cells; the albumin gene promoter, the Apo AI and Apo AII control regions active in liver, the alpha-fetoprotein gene control region active in liver, the alpha 1-antitrypsin gene control region active in the liver, the beta-globin gene control region active in myeloid cells, the myelin basic protein gene control region active in oligodendrocyte cells in the brain, the myosin light chain-2 gene control region active in skeletal muscle, and the gonadotropic releasing hormone gene control region active in the hypothalamus, the pyruvate kinase promoter, the villin promoter, the promoter of the fatty acid binding intestinal protein, the promoter of smooth muscle cell α-actin, and the like. In addition, any of these expression sequences of this invention can be modified by addition of enhancer and/or regulatory sequences and the like.

Enhancers that may be used in embodiments of the invention include but are not limited to: an SV40 enhancer, a cytomegalovirus (CMV) enhancer, an elongation factor I (EF1) enhancer, yeast enhancers, viral gene enhancers, and the like.

Termination control regions, *i.e.,* terminator or polyadenylation sequences, may be derived from various genes native to the preferred hosts. In some embodiments of the invention, the termination control region may comprise or be derived from a synthetic sequence, a synthetic polyadenylation signal, an SV40 late polyadenylation signal, an SV40 polyadenylation signal, a bovine growth hormone (BGH) polyadenylation signal, viral terminator sequences, or the like.

It will be apparent to those skilled in the art that any suitable vector can be used to deliver the polypeptide and/or polynucleotide to a cell or subject. The vector can be delivered to cells *in vivo.* **In** other embodiments, the vector can be delivered to cells *ex vivo,* and then cells containing the vector are delivered to the subject. The choice of delivery vector can be made based on a number of factors known in the art, including age and species of the target host, *in vitro* versus *in vivo* delivery, level and persistence of expression desired, intended purpose *(e.g.,* for therapy or screening), the target cell or organ, route of delivery, size of the isolated polynucleotide, safety concerns, and the like.

The vector can be a commercial expression vector or it can be constructed in the laboratory according to standard molecular biology protocols. The vector can comprise viral nucleic acid including, but not limited to, poxvirus, vaccinia virus, adenovirus, retrovirus, alphavirus and/or adeno-associated virus nucleic acid. The nucleic acid molecule or vector of this invention can also be in a liposome or a delivery vehicle, which can be taken up by a cell via receptor-mediated or other type of endocytosis. The nucleic acid molecule of this invention can be in a cell, which can be a cell expressing the nucleic acid whereby a synthetic polynucleotide of this invention is produced in the cell (e.g., a host cell). In addition, the vector of this invention can be in a cell, which can be a cell expressing the nucleic acid of the vector whereby a synthetic polynucleotide of this invention is produced in the cell. It is also contemplated that the nucleic acid molecules and/or vectors of this invention can be present in a host organism (e.g., a transgenic organism), which expresses the nucleic acids of this invention and produces the synthetic polynucleotide of this invention. Suitable vectors include plasmid vectors, viral vectors (e.g., retrovirus, alphavirus; vaccinia virus; adenovirus, adeno-associated virus and other parvoviruses, lentivirus, poxvirus, or herpes simplex virus), lipid vectors, poly-lysine vectors, synthetic polyamino polymer vectors, and the like. Any viral vector that is known in the art can be used in the present invention. Protocols for producing recombinant viral vectors and for using viral vectors for nucleic acid delivery can be found in Ausubel et al., Current Protocols in Molecular Biology (Green Publishing Associates, Inc. and John Wiley & Sons, Inc., New York) and other standard laboratory manuals *(e.g.,* Vectors for Gene Therapy. In: Current Protocols in Human Genetics. John Wiley and Sons, Inc.: 1997). In some embodiments, the vector is a plasmid, a viral vector, a bacterial vector, an expression cassette, a transformed cell, or a nanoparticle.

Non-viral transfer methods can also be employed. Many non-viral methods of nucleic acid transfer rely on normal mechanisms used by mammalian cells for the uptake and intracellular transport of macromolecules. In particular embodiments, non-viral nucleic acid delivery systems rely on endocytic pathways for the uptake of the nucleic acid molecule by the targeted cell. Exemplary nucleic acid delivery systems of this type include liposomal derived systems, poly-lysine conjugates, and artificial viral envelopes.

In some embodiments, plasmid vectors are used in the practice of the present invention. For example, naked plasmids can be introduced into muscle cells by injection into the tissue. Expression can extend over many months, although the number of positive cells is typically low (Wolff et al., Science 247:247 (1989)). Cationic lipids have been demonstrated to aid in introduction of nucleic acids into some cells in culture (Felgner and Ringold, Nature 337:387 (1989)). Injection of cationic lipid plasmid DNA complexes into the circulation of mice has been shown to result in expression of the DNA in lung (Brigham et al., Am. J. Med. Sci. 298:278 (1989)). One advantage of plasmid DNA is that it can be introduced into non-replicating cells.

In a representative embodiment, a nucleic acid molecule *(e.g.,* a plasmid) can be entrapped in a lipid particle bearing positive charges on its surface and, optionally, tagged with antibodies against cell surface antigens of the target tissue (Mizuno et al., No Shinkei Geka 20:547 (1992); PCT publication WO 91/06309; Japanese patent application 1047381; and European patent publication EP-A-43075).

Liposomes that consist of amphiphilic cationic molecules are useful as non-viral vectors for nucleic acid delivery *in vitro* and *in vivo* (reviewed in Crystal, Science 270:404 (1995); Blaese et al., Cancer Gene Ther. 2:291 (1995); Behr et al., Bioconjugate Chem. 5:382 (1994); Remy et al., Bioconjugate Chem. 5:647 (1994); and Gao et al., Gene Therapy 2:710 (1995)). The positively charged liposomes are believed to complex with negatively charged nucleic acids via electrostatic interactions to form lipid:nucleic acid complexes. The lipid:nucleic acid complexes have several advantages as nucleic acid transfer vectors. Unlike viral vectors, the lipid:nucleic acid complexes can be used to transfer expression cassettes of essentially unlimited size. Since the complexes lack proteins, they can evoke fewer immunogenic and inflammatory responses. Moreover, they cannot replicate or recombine to form an infectious agent and have low integration frequency. A number of publications have demonstrated that amphiphilic cationic lipids can mediate nucleic acid delivery *in vivo* and *in vitro* (Felgner et al., Proc. Natl. Acad. Sci. USA 84:7413 (1987); Loeffler et al., Meth. Enzymol. 217:599 (1993); Felgner et al., J. Biol. Chem. 269:2550 (1994)).

Viral vectors have been used in a wide variety of gene delivery applications in cells, as well as living animal subjects. Viral vectors that can be used include, but are not limited to, retrovirus, lentivirus, adeno-associated virus, poxvirus, alphavirus, baculovirus, vaccinia virus, herpes virus, Epstein-Barr virus, adenovirus, geminivirus, and caulimovirus vectors. Non-viral vectors include plasmids, liposomes, electrically charged lipids (cytofectins), nucleic acid-protein complexes, and biopolymers. In addition to a nucleic acid of interest, a vector may also comprise one or more regulatory regions, and/or selectable markers useful in selecting, measuring, and monitoring nucleic acid transfer results (delivery to specific tissues, duration of expression, *etc.).*

In addition to the regulatory control sequences discussed above, the recombinant expression vector can contain additional nucleotide sequences. For example, the recombinant expression vector can encode a selectable marker gene to identify host cells that have incorporated the vector.

Vector DNA can be introduced into prokaryotic or eukaryotic cells via conventional transformation or transfection techniques. As used herein, the terms "transformation" and "transfection" refer to a variety of art-recognized techniques for introducing foreign nucleic acids *(e.g.,* DNA and RNA) into a host cell, including calcium phosphate or calcium chloride co-precipitation, DEAE-dextran-mediated transfection, lipofection, electroporation, microinjection, DNA-loaded liposomes, lipofectamine-DNA complexes, cell sonication, gene bombardment using high velocity microprojectiles, and viral-mediated transfection. Suitable methods for transforming or transfecting host cells can be found in Sambrook et al., Molecular Cloning: A Laboratory Manual 2nd Ed. (Cold Spring Harbor, NY, 1989), and other laboratory manuals.

If stable integration is desired, often only a small fraction of cells (in particular, mammalian cells) integrate the foreign DNA into their genome. In order to identify and select integrants, a nucleic acid that encodes a selectable marker (e.g., resistance to antibiotics) can be introduced into the host cells along with the nucleic acid of interest. Preferred selectable markers include those that confer resistance to drugs, such as G418, hygromycin and methotrexate. Nucleic acids encoding a selectable marker can be introduced into a host cell on the same vector as that comprising the nucleic acid of interest or can be introduced on a separate vector. Cells stably transfected with the introduced nucleic acid can be identified by drug selection (e.g., cells that have incorporated the selectable marker gene will survive, while the other cells die).

A further aspect of the invention relates to a composition comprising the synthetic polypeptide of the invention and a carrier. In some embodiments, the composition is a pharmaceutical composition comprising the synthetic polypeptide of the invention and a pharmaceutically acceptable carrier.

By "pharmaceutically acceptable" it is meant a material that is not toxic or otherwise undesirable, i.e., the material may be administered to a subject without causing any undesirable biological effects. For injection, the carrier will typically be a liquid. For other methods of administration (e.g., such as, but not limited to, administration to the mucous membranes of a subject (e.g., via intranasal administration, buccal administration and/or inhalation)), the carrier may be either solid or liquid. For inhalation administration, the carrier will be respirable, and will preferably be in solid or liquid particulate form. The formulations may be conveniently prepared in unit dosage form and may be prepared by any of the methods well known in the art. In some embodiments, that pharmaceutically acceptable carrier can be a sterile solution or composition.

In some embodiments, the present invention provides a pharmaceutical composition comprising the synthetic polypeptide, nucleic acid molecule, vector, and/or composition of the present invention, a pharmaceutically acceptable carrier, and optionally, other medicinal agents, therapeutic agents, pharmaceutical agents, stabilizing agents, buffers, carriers, adjuvants, diluents, etc., which can be included in the composition singly or in any combination and/or ratio.

In some embodiments, a pharmaceutical composition of the present invention may further comprise a therapeutic agent selected from, but not limited to, the group consisting of dexamethasone, remdesivir, elexacaftor, ivacaftor, lumacaftor, tezacaftor, ibuprofen, acebilustat, lenabasum, levofloxacin, piperacillin-taxobactam, vancomycin, azithromycin, ciprofloxacin, cephalexin, doxycycline hyclate, and any combination thereof.

Immunogenic compositions comprising a synthetic polypeptide, nucleic acid molecule, vector, and/or composition of the present invention may be formulated by any means known in the art. Such compositions are typically prepared as injectables, either as liquid solutions or suspensions. Solid forms suitable for solution in, or suspension in, liquid prior to injection may also be prepared. Lyophilized preparations are also suitable. The active immunogenic ingredients are often mixed with excipients and/or carriers that are pharmaceutically acceptable and/or compatible with the active ingredient. Suitable excipients include but are not limited to sterile water, saline, dextrose, glycerol, ethanol, or the like and combinations thereof, as well as stabilizers, e.g., HSA or other suitable proteins and reducing sugars. In addition, if desired, immunogenic compositions may contain minor amounts of auxiliary substances such as wetting and/or emulsifying agents, pH buffering agents, and/or adjuvants that enhance the effectiveness of the vaccine or immunogenic composition.

Additionally provided here is a dosage delivery device comprising a pharmaceutical composition of the present invention. In some embodiments, the dosage delivery device is an inhaler, e.g., for delivery of the composition to the airways of a subject, e.g., by oral and/or nasal inhalation.

Another aspect of the invention relates to a kit comprising the synthetic polypeptide, nucleic acid molecule, vector, cell, and/or composition of the invention and useful for carrying out the methods of the invention. The kit may further comprise additional reagents for carrying out the methods (e.g., buffers, containers, additional therapeutic agents) as well as instructions.

### Methods

The *in vitro* methods and medical uses of the invention relate to the ability of synthetic polypeptides to bind to an Orai1 calcium channel, e.g., a human Orai1 calcium channel.

Thus, one aspect of the present invention provides an *in vitro* method of inhibiting calcium influx through a calcium channel, comprising contacting the calcium channel with a synthetic polypeptide or a pharmaceutical composition of the present invention, thereby inhibiting calcium influx through the calcium channel.

The inhibition of calcium channel activity can be measured by any method known in the art or disclosed herein, including, without limitation, measuring calcium flow or change in potential across a membrane, across a cell, or across a natural or artificial lining. The inhibition can be at least about 20%, *e.g.,* at least about 30%, 40%, 50%, 60%, 70%, 80%, 90%, or 100%.

Another aspect of the invention relates to a synthetic polypeptide of the invention for use in a method of inhibiting airway smooth muscle contraction, comprising contacting the airway with the polypeptide of the invention, thereby inhibiting airway smooth muscle contraction. Inhibition of smooth muscle contraction can be measured by any technique known in the art or disclosed herein. Inhibition of contraction is measured relative to the level of contraction in the absence of contact with the polypeptide of the invention. In some embodiments, contraction is inhibited by at least about 10%, *e.g.,* at least about 10%, 20%, 30%, 40%, 50%, 60%, 70%, 80%, 90%, or more.

Another aspect of the invention relates to a synthetic polypeptide of the invention for use in a method of inhibiting airway hyperreactivity, comprising contacting the airway with the polypeptide of the invention, thereby inhibiting airway hyperreactivity. Airway reactivity can be measured by any technique known in the art or disclosed herein. Inhibition of hyperreactivity is measured relative to the level of reactivity in the absence of contact with the polypeptide of the invention. "Hyperreactivity," as used herein, refers to increased reactivity of the airway to calcium relative to the level of reactivity of normal airway. In some embodiments, hyperreactivity is inhibited by at least about 10%, *e.g.,* at least about 10%, 20%, 30%, 40%, 50%, 60%, 70%, 80%, 90%, or more.

Another aspect of the present invention provides an *in vitro* method of reducing expression of an Orai1 calcium channel, comprising contacting the calcium channel Orai1 with a synthetic polypeptide or a pharmaceutical composition of the present invention. While not wishing to be bound by theory, the synthetic polypeptides of the invention may induce allostery in Orai1 that causes Orai1 to be internalized and degraded, e.g., in the cell.

The *in vitro* methods of the present invention can be carried out, *e.g.,* on an isolated Orai1 calcium channel, an Orai1 calcium channel in an artificial membrane, or an Orai1 calcium channel in a cell. In one embodiment, the calcium channel is present in an isolated cell, *e.g.,* a cultured primary cell or cell line. In certain embodiments, the cell is an airway smooth muscle cell, e.g., part of an airway smooth muscle cell culture. In another embodiment, the isolated cell is part of an epithelial cell culture, *e.g.,* a natural or artificial epithelial lining, *e.g.,* a cell culture in a device (such as an Ussing chamber) in which characteristics such as ion flow and/or potential can be measured across a lining. In another embodiment, the isolated cell is an immune system cell, *e.g.,* a leukocyte, lymphocyte, T cell, mast cell, macrophage, *etc.* In another embodiment, the isolated cell is a cancer cell. In another embodiment, the cell is part of an isolated tissue or a tissue culture.

In some embodiments, contacting the calcium channel with the synthetic polypeptide in an animal inhibits calcium influx only in the lungs (e.g., does not reduce global Ca²⁺) of the animal. In some embodiments, the animal is a disease model, e.g., a murine disease model.

In some embodiments, contacting the calcium channel with the synthetic polypeptide comprises delivering the synthetic polypeptide to a cell comprising the calcium channel.

In some embodiments, contacting the calcium channel with the synthetic polypeptide comprises delivering a polynucleotide encoding the synthetic polypeptide to a cell comprising the calcium channel.

In some embodiments, contacting the calcium channel with the synthetic polypeptide comprises delivering the synthetic polypeptide directly to the lungs (e.g., administering the synthetic polypeptide, polynucleotide, vector, and/or composition (e.g., pharmaceutical composition) of the present invention directly to the lungs), optionally by intranasal, intratracheal, inhalation, and/or intrapulmonary administration).

In some embodiments, contacting the calcium channel with the synthetic polypeptide comprises delivering the synthetic polypeptide to a target organ system (e.g., administering the synthetic polypeptide, polynucleotide, vector, and/or composition of the present invention to a target organ system), optionally systemically, optionally by intravenous administration.

Target organ systems contemplated in the present invention may be any mammalian organ system to which the compositions and methods of the invention may provide benefit. Non-limiting examples of target organ systems include the nervous system (e.g., PNS, CNS), the muscular system, the respiratory system (e.g., the lungs, e.g., the airways), the reproductive system, the lymphatic system, the renal system, the digestive system, systemically, or any combination thereof. In some embodiments, the target organ system is the lungs (e.g., the airways). In some embodiments, the target organ system is the organism (e.g., systemically).

Another aspect of the present invention provides the synthetic polypeptide, the polynucleotide, the vector, and/or the composition, of the invention, for use in a method of inhibiting an immune response in a subject, comprising delivering to the subject a synthetic polypeptide, polynucleotide, vector, and/or composition (e.g., pharmaceutical composition) of the present invention, thereby inhibiting the immune response. In some embodiments, the immune response is inhibited by at least about 10%, *e.g.,* at least about 10%, 25%, 50%, 75%, or more. Inhibition of the immune response can be quantitated by methods known in the art, *e.g.,* by measurement of the level of immune system cells and/or antibodies in the blood or in the tissue of a subject.

Another aspect of the present invention provides the synthetic polypeptide, the polynucleotide, the vector, and/or the composition, of the invention, for use in a method of inhibiting inflammation in a subject, comprising delivering to the subject a synthetic polypeptide, polynucleotide, vector, and/or composition (e.g., pharmaceutical composition) of the present invention, thereby inhibiting the inflammation. In some embodiments, inflammation is inhibited by at least about 10%, *e.g.,* at least about 10%, 25%, 50%, 75%, or more. Inhibition of inflammation can be quantitated by methods known in the art, *e.g.,* by measurement of the level of immune system cells, interleukins, chemokines, or other biological effector molecules in the blood or in the tissue of a subject.

In some embodiments, delivering to the subject a synthetic polypeptide, polynucleotide, vector, and/or composition (e.g., pharmaceutical composition) of the present invention may comprise delivering the synthetic polypeptide, polynucleotide, vector, and/or composition (e.g., pharmaceutical composition) directly to the respiratory target organ system (e.g., the lungs, the airways), optionally via intranasal, intratracheal, inhalation, and./or intrapulmonary administration/delivery.

In some embodiments, delivering to the subject a synthetic polypeptide, polynucleotide, vector, and/or composition (e.g., pharmaceutical composition) of the present invention may comprise delivering the synthetic polypeptide, polynucleotide, vector, and/or composition (e.g., pharmaceutical composition) to a target organ system, optionally systemically, optionally via intravenous administration/delivery.

Another aspect of the present invention provides the synthetic polypeptide, the polynucleotide, the vector, and/or the composition, of the invention, for use in a method of treating or preventing a disorder responsive to inhibition of calcium influx in an airway in a subject in need thereof, comprising delivering to the airway of the subject a synthetic polypeptide, polynucleotide, vector, and/or composition (e.g., pharmaceutical composition) of the present invention, thereby treating or preventing the disorder. As used herein, the term "disorder responsive to inhibition of calcium influx in an airway," refers to any disease, disorder, or condition that can be treated and/or prevented by inhibiting calcium influx in an airway. The disorder in the methods of the invention can be, in non-limiting examples, asthma or respiratory allergies. In certain embodiments, the polypeptide is delivered by inhalation, *e.g.*, using an inhaler or nebulizer for delivery by oral and/or nasal inhalation. In some embodiments, the polypeptide is delivered to the airway of the subject via intranasal, intratracheal, intrapulmonary, and/or inhalation delivery.

Disorders of application by the methods of the present invention may include, but are not limited to, inflammatory lung disorders such as asthma, chronic obstructive pulmonary disease, idiopathic pulmonary fibrosis, cystic fibrosis, non-cystic fibrosis bronchiectasis, and acute or chronic bronchitis. In some embodiments, the disorder is asthma. In some embodiments, the disorder is chronic obstructive pulmonary disease ((COPD), e.g., chronic bronchitis or emphysema). In some embodiments, the disorder is an allergy (e.g., a dust allergy, pollen allergy, mold allergy (e.g., *Aspergillus fumigatus*)*,* etc.

In some embodiments, the disorder may be a genetic inflammatory condition of the lungs. In some embodiments, the disorder may be cystic fibrosis.

In some embodiments, the disorder may be fibrosis, such as, but not limited to, idiopathic pulmonary fibrosis.

In some embodiments, the disorder may pneumonia, such as, but not limited to, ventilator associated pneumonia (VAP), hospital acquired pneumonia (HAP), viral associated pneumonia and/or bacterial associated pneumonia or COVID-19.

In some embodiments, the disorder may be an infection, such as viral, bacterial, and/or fungal infection. Thus, in some embodiments, the infection may be a respiratory infection wherein the infectious agent is *S. aureus,* methicillin-resistant *Staphylococcus aureus* (MRSA), *P. aeruginosa, H. influenzae, K. pneumonia, E. coli, Acinetobacter, Enterobacter, S. pneumoniae, M. catarrhalis, M. pneumoniae, mycobacterium, mycoplasma pneumonia,* fungi (e.g., *Aspergillus fumigates, Cryptococcus, Pneumocystis, Candida),* or any combination thereof. In some embodiments, the infection may be a viral respiratory infection wherein the infectious agent is a coronavirus (e.g., MHV-A59, SARS CoV, SARS-CoV-2, or MERS-CoV), dengue virus, hepatitis virus, influenza virus (e.g., influenza A, influenza B), respiratory syncytial virus (RSV), rhinovirus, parainfluenza virus, metapneumovirus, or any other respiratory virus or any combination thereof.

Another aspect of the invention provides the synthetic polypeptide, the polynucleotide, the vector, and/or the composition, of the invention, for use in a method of treating or preventing a disorder responsive to inhibition of calcium influx in a subject in need thereof, comprising delivering to the subject a therapeutically or prophylactically effective amount of a synthetic polypeptide, polynucleotide, vector, and/or composition (e.g., pharmaceutical composition) of the present invention, thereby treating or preventing the disorder. In some embodiments, the synthetic polypeptide, polynucleotide, vector, and/or composition of the present invention may be delivered directly to a target organ system. In some embodiments, the synthetic polypeptide, polynucleotide, vector, and/or composition of the present invention may be delivered directly to the subject, e.g., systemically, optionally via intravenous delivery.

Accordingly, further disorders applicable to the present invention include, but are not limited to, acute pancreatitis, arthritis, allergy, Bell's Palsy, cardiovascular disease (CVD; e.g., including but not limited to vasculitis), dermatomyositis, diabetes, Guillain-Barré Syndrome, inflammatory bowel disease (e.g., Ulcerative Colitis And Crohn's Disease), Lupus, Myasthenia Gravis, psoriasis, reactive arthritis, renal inflammation, rheumatoid arthritis, sarcoidosis, scleroderma, infection (e.g., sepsis), Sjögren's Syndrome, temporal arteritis, Kawasaki disease, or any combination thereof.

In some embodiments, the disorder may be an infection, e.g., an infection in a particular target organ system and/or a systemic infection (e.g., sepsis). In some embodiments, the infection may be a bacterial, viral, and/or fungal infection. In some embodiments, the disorder may be sepsis.

In some embodiments, the infection may be a systemic and/or local target organ system infection wherein the infectious agent is bacterial (e.g., *S. aureus,* methicillin-resistant *Staphylococcus aureus* (MRSA), *P. aeruginosa, H. influenzae, K. pneumonia, E. coli, Acinetobacter, Enterobacter, S. pneumoniae, M. catarrhalis, M. pneumoniae, mycobacterium, mycoplasma pneumonia, group B streptococci, Neisseria meningitidis),* fungal (e.g., *Aspergillus fumigates, Cryptococcus, Pneumocystis, Candida),* viral (e.g., coronavirus (e.g., MHV-A59, SARS CoV, SARS-CoV-2, or MERS-CoV), dengue virus, hepatitis virus, influenza virus (e.g., influenza A, influenza B), respiratory syncytial virus (RSV), rhinovirus, parainfluenza virus, metapneumovirus), or any combination thereof.

In some embodiments, the synthetic polypeptide, polynucleotide, vector, and/or composition (e.g., pharmaceutical composition) of the present invention may be delivered/administered systemically to the subject via intravenous delivery/administration.

In some embodiments, delivering the synthetic polypeptide, polynucleotide, vector, and/or composition (e.g., pharmaceutical composition) of the present invention to the subject does not inhibit calcium influx in the lungs of the subject.

In some embodiments of the present invention, the polypeptides of the invention are administered directly to a subject. Generally, the compounds of the invention will be suspended in a pharmaceutically-acceptable carrier (e.g., physiological saline) and administered orally or by intravenous infusion, or administered subcutaneously, intramuscularly, intrathecally, intraperitoneally, intrarectally, intravaginally, intranasally, intragastrically, intratracheally, or intrapulmonarily. In another embodiment, the intratracheal or intrapulmonary delivery can be accomplished using a standard nebulizer, jet nebulizer, wire mesh nebulizer, dry powder inhaler, or metered dose inhaler. They can be delivered directly to the site of the disease or disorder, such as lungs, kidney, or intestines. The dosage required depends on the choice of the route of administration; the nature of the formulation; the nature of the patient's illness; the subject's size, weight, surface area, age, and sex; other drugs being administered; and the judgment of the attending physician. Suitable dosages are in the range of 0.01-100.0 µg/kg.

In some embodiments, the synthetic polypeptide is administered/delivered in an amount of about 0.1 mg/kg to about 10 mg/kg of the subject, e.g., about 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1 mg/kg, 1.25 mg/kg, 1.5 mg/kg, 1.75 mg/kg, 2 mg/kg, 3 mg/kg, 4 mg/kg, 5 mg/kg, 6 mg/kg, 7 mg/kg, 8 mg/kg, 9 mg/kg, or 10 mg/kg or any value or range therein. For example, in some embodiments, the synthetic polypeptide of the present invention may be administered/ delivered in an amount of about 0.1 mg/kg to about 9.75 mg/kg, about 2 mg/kg to about 5 mg/kg, about 0.25 mg/kg to about 2.5 mg/kg, about 5 mg/kg to about 10 mg/kg, or about 0.50 mg/kg to about 1 mg/kg of the subject. In some embodiments, the synthetic polypeptides of the present invention may be administered/delivered in an amount of about 0.1 mg/kg, about 0.25 mg/kg, about 0.50 mg/kg, about 1 mg/kg, or about 4 mg/kg of the subject.

Wide variations in the needed dosage are to be expected in view of the variety of polypeptides available and the differing efficiencies of various routes of administration. The synthetic polypeptide of the present invention and/or a nucleic acid molecule, vector, and/or pharmaceutical composition comprising the same may be delivered in any number of doses and according to any regimen or schedule as needed to achieve the desired therapeutic effect. For example, oral administration would be expected to require higher dosages than administration by i.v. injection. Variations in these dosage levels can be adjusted using standard empirical routines for optimization as is well understood in the art. Administrations can be single or multiple *(e.g.,* 2-, 3-, 4-, 6-, 8-, 10-; 20-, 50-, 100-, 150-, or more fold. For example, the synthetic polypeptide may be delivered in one or more doses, e.g., two or more, three or more, four or more doses, etc., or in other words, at least one, at least two, at least three, at least four or more doses, etc. In some embodiments, a single dose of the synthetic polypeptide is delivered. In some embodiments, multiple doses of the synthetic polypeptide are delivered.

In some embodiments, a synthetic polypeptide of the present invention may be delivered according to a regimen/schedule of continual dosing over an extended period of time (e.g., chronic dosing). Aspects of an appropriate regimen/schedule of dosing may be ascertainable by a person of skill in the art, e.g., a physician, and adjusted according to relevant parameters of the subject and disorder such as noted above.

Encapsulation of the polypeptides in a suitable delivery vehicle (e.g., polymeric microparticles or implantable devices) may increase the efficiency of delivery, particularly for oral delivery.

In some embodiments, the synthetic polypeptide is delivered by inhalation.

In some embodiments, delivering the synthetic polypeptide or pharmaceutical composition to the subject inhibits calcium influx only in the lungs (e.g., does not reduce global Ca²⁺) of the subject.

The polypeptide of the present invention can optionally be delivered in conjunction with other therapeutic agents. The additional therapeutic agents can be delivered concurrently with the polypeptide of the invention. As used herein, the word "concurrently" means sufficiently close in time to produce a combined effect (that is, concurrently can be simultaneously, or it can be two or more events occurring within a short time period before or after each other). Thus, in some embodiments, the medical uses of the present invention may further comprise delivering to the subject a therapeutic agent as known in the art for the treatment of the disorders of the invention. In one embodiment of the invention, the polypeptide is delivered to a patient concurrently with a compound that treats and/or prevents asthma, e.g., a bronchodilator such as a β agonist or a steroid. In other embodiments, the is delivered to a patient concurrently with a compound that treats and/or prevents allergies, *e.g.,* an antihistamine. In some embodiments, the combined activity of the polypeptide and the other therapeutic agent is superior to the other therapeutic agent alone.

Additional therapeutic agents as known in the art for the treatment of the disorders of the invention which may be delivered in conjunction with a polypeptide of the present invention include, but not limited to, dexamethasone, corticosteroids (e.g., Fluticasone (Flovent HFA), Budesonide (Pulmicort Flexhaler), Mometasone (Asmanex Twisthaler), Beclomethasone (Qvar RediHaler), Ciclesonide (Alvesco)); remdesivir (e.g., for COVID19); elexacaftor, ivacaftor, lumacaftor, tezacaftor, ibuprofen, acebilustat, lenabasum (e.g., for cystic fibrosis); hypertonic saline, dornase alfa (Pulmozyme^{®}), amikacin, amoxicillin, augmentin, ampicillin, bactrim (sulfamethoxazole and/or trimethoprim) levofloxacin, doxycycline, tetracycline, piperacillin-taxobactam, vancomycin, clindamycin, azithromycin, clarithromycin, ciprofloxacin (e.g., for pneumonia such as HAP and/or VAP), cephalexin, doxycycline hyclate, mepolizumab, reslizumab, Lebrikizumab, Tralokinumab, Tezepelumab, benralizumab, and any other antibiotic known for the treatment of pneumonia.

As a further aspect, the invention provides pharmaceutical formulations, and a pharmaceutical formulation for use in a method of administering said pharmaceutical formulation, the same to achieve any of the therapeutic effects (e.g., modulation of calcium influx) discussed above. The pharmaceutical formulation may comprise any of the reagents discussed above in a pharmaceutically acceptable carrier, *e.g.,* a polypeptide and/or nucleic acid and/or vector comprising the same. By "pharmaceutically acceptable" it is meant a material that is not biologically or otherwise undesirable, *i.e.,* the material can be administered to a subject without causing any undesirable biological effects such as toxicity.

The formulations of the invention can optionally comprise medicinal agents, pharmaceutical agents, carriers, adjuvants, dispersing agents, diluents, and the like.

The peptides of the invention can be formulated for administration in a pharmaceutical carrier in accordance with known techniques. *See, e.g.,* Remington, The Science And Practice of Pharmacy (9th Ed. 1995). In the manufacture of a pharmaceutical formulation according to the invention, the polypeptide (including the physiologically acceptable salts thereof) is typically admixed with, *inter alia,* an acceptable carrier. The carrier can be a solid or a liquid, or both, and may be formulated with the polypeptide as a unit-dose formulation, for example, a metered dose inhaler, which can contain from 0.01 or 0.5% to 95% or 99% by weight of the polypeptide. One or more polypeptide or a functional fragment or homolog thereof can be incorporated in the formulations of the invention, which can be prepared by any of the well-known techniques of pharmacy.

A further aspect of the invention is the synthetic polypeptide, the polynucleotide, the vector, and/or the composition, of the invention, for use in a method of treating subjects *in vivo,* comprising administering to a subject a pharmaceutical composition comprising a polypeptide of the invention in a pharmaceutically acceptable carrier, wherein the pharmaceutical composition is administered in a therapeutically effective amount. Administration of the polypeptide of the present invention to a human subject or an animal in need thereof can be by any means known in the art for administering compounds.

The formulations of the invention include those suitable for oral, rectal, topical, buccal *(e.g.,* sub-lingual), vaginal, parenteral *(e.g.,* subcutaneous, intramuscular including skeletal muscle, cardiac muscle, diaphragm muscle and smooth muscle, intradermal, intravenous, intraperitoneal), topical *(i.e.,* both skin and mucosal surfaces, including airway surfaces), intranasal, transdermal, intraarticular, intrathecal, and inhalation administration, administration to the liver by intraportal delivery, as well as direct organ injection (e.g., into the liver, into the brain for delivery to the central nervous system, into the pancreas, or into a tumor or the tissue surrounding a tumor). The most suitable route in any given case will depend on the nature and severity of the condition being treated and on the nature of the particular peptide which is being used.

For injection, the carrier will typically be a liquid, such as sterile pyrogen-free water, sterile normal saline, hypertonic saline, pyrogen-free phosphate-buffered saline solution, bacteriostatic water, or Cremophor EL[R] (BASF, Parsippany, N.J.). For other methods of administration, the carrier can be either solid or liquid.

For oral administration, the polypeptide can be administered in solid dosage forms, such as capsules, tablets, and powders, or in liquid dosage forms, such as elixirs, syrups, and suspensions. Polypeptides can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that can be added to provide desirable color, taste, stability, buffering capacity, dispersion or other known desirable features are red iron oxide, silica gel, sodium lauryl sulfate, titanium dioxide, edible white ink and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric- coated for selective disintegration in the gastrointestinal tract. Liquid dosage forms for oral administration can contain coloring and flavoring to increase patient acceptance.

Formulations suitable for buccal (sub-lingual) administration include lozenges comprising the compound in a flavored base, usually sucrose and acacia or tragacanth; and pastilles comprising the compound in an inert base such as gelatin and glycerin or sucrose and acacia.

Formulations of the present invention suitable for parenteral administration comprise sterile aqueous and non-aqueous injection solutions of the polypeptide, which preparations are preferably isotonic with the blood of the intended recipient. These preparations can contain antioxidants, buffers, bacteriostats and solutes which render the formulation isotonic with the blood of the intended recipient. Aqueous and non-aqueous sterile suspensions can include suspending agents and thickening agents. The formulations can be presented in unit/dose or multi-dose containers, for example sealed ampoules and vials, and can be stored in a freeze-dried (lyophilized) condition requiring only the addition of the sterile liquid carrier, for example, saline or water-for-injection immediately prior to use.

Extemporaneous injection solutions and suspensions can be prepared from sterile powders, granules and tablets of the kind previously described. For example, in one aspect of the present invention, there is provided an injectable, stable, sterile composition comprising a polypeptide or a functional fragment or homolog thereof of the invention, in a unit dosage form in a sealed container. The polypeptide or salt is provided in the form of a lyophilizate which is capable of being reconstituted with a suitable pharmaceutically acceptable carrier to form a liquid composition suitable for injection thereof into a subject. The unit dosage form typically comprises from about 1 mg to about 10 grams of the polypeptide or salt. When the polypeptide or salt is substantially water-insoluble, a sufficient amount of emulsifying agent which is pharmaceutically acceptable can be employed in sufficient quantity to emulsify the peptide or salt in an aqueous carrier. One such useful emulsifying agent is phosphatidyl choline.

Formulations suitable for rectal administration are preferably presented as unit dose suppositories. These can be prepared by admixing the polypeptide with one or more conventional solid carriers, for example, cocoa butter, and then shaping the resulting mixture.

Formulations suitable for topical application to the skin preferably take the form of an ointment, cream, lotion, paste, gel, spray, aerosol, or oil. Carriers which can be used include petroleum jelly, lanoline, polyethylene glycols, alcohols, transdermal enhancers, and combinations of two or more thereof.

Formulations suitable for transdermal administration can be presented as discrete patches adapted to remain in intimate contact with the epidermis of the recipient for a prolonged period of time. Formulations suitable for transdermal administration can also be delivered by iontophoresis *(see,* for example, Tyle, Pharm. Res. 3:318 (1986)) and typically take the form of an optionally buffered aqueous solution of the polypeptide. Suitable formulations comprise citrate or bis/tris buffer (pH 6) or ethanol/water and contain from 0.1 to 0.2M of the compound.

The polypeptide can alternatively be formulated for nasal administration or otherwise administered to the lungs of a subject by any suitable means, e.g., administered by an aerosol suspension of respirable particles comprising the polypeptide, which the subject inhales. The respirable particles can be liquid or solid. The term "aerosol" includes any gas-borne suspended phase, which is capable of being inhaled into the bronchioles or nasal passages. Specifically, aerosol includes a gas-borne suspension of droplets, as can be produced in a metered dose inhaler or nebulizer, or in a mist sprayer. Aerosol also includes a dry powder composition suspended in air or other carrier gas, which can be delivered by insufflation from an inhaler device, for example. *See* Ganderton & Jones, Drug Delivery to the Respiratory Tract, Ellis Horwood (1987); Gonda (1990) Critical Reviews in Therapeutic Drug Carrier Systems 6:273-313; and Raeburn et al., J. Pharmacol. Toxicol. Meth. 27:143 (1992). Aerosols of liquid particles comprising the polypeptide can be produced by any suitable means, such as with a pressure-driven aerosol nebulizer or an ultrasonic nebulizer, as is known to those of skill in the art. *See, e.g.,* U.S. Patent No. 4,501,729. Aerosols of solid particles comprising the polypeptide can likewise be produced with any solid particulate medicament aerosol generator, by techniques known in the pharmaceutical art.

Alternatively, one can administer the polypeptide in a local rather than systemic manner, for example, in a depot or sustained-release formulation.

Further, the present invention provides liposomal formulations of the polypeptide disclosed herein and salts thereof. The technology for forming liposomal suspensions is well known in the art. When the polypeptide or salt thereof is an aqueous-soluble salt, using conventional liposome technology, the same can be incorporated into lipid vesicles. In such an instance, due to the water solubility of the polypeptide or salt, the polypeptide or salt will be substantially entrained within the hydrophilic center or core of the liposomes. The lipid layer employed can be of any conventional composition and can either contain cholesterol or can be cholesterol-free. When the polypeptide or salt of interest is water-insoluble, again employing conventional liposome formation technology, the salt can be substantially entrained within the hydrophobic lipid bilayer which forms the structure of the liposome. In either instance, the liposomes which are produced can be reduced in size, as through the use of standard sonication and homogenization techniques.

The liposomal formulations containing the polypeptide disclosed herein or salts thereof, can be lyophilized to produce a lyophilizate which can be reconstituted with a pharmaceutically acceptable carrier, such as water, to regenerate a liposomal suspension.

In the case of a water-insoluble polypeptide or a functional fragment or homolog thereof, a pharmaceutical composition can be prepared containing the water-insoluble polypeptide or a functional fragment or homolog thereof, such as for example, in an aqueous base emulsion. In such an instance, the composition will contain a sufficient amount of pharmaceutically acceptable emulsifying agent to emulsify the desired amount of the polypeptide. Particularly useful emulsifying agents include phosphatidyl cholines and lecithin.

In particular embodiments, the polypeptide is administered to the subject in a therapeutically effective amount, as that term is defined above. Dosages of pharmaceutically active polypeptides can be determined by methods known in the art, *see, e.g.,* Remington's Pharmaceutical Sciences (Mack Publishing Co., Easton, Pa). The therapeutically effective dosage of any specific polypeptide or a functional fragment or homolog thereof will vary somewhat from compound to compound, and patient to patient, and will depend upon the condition of the patient and the route of delivery. As a general proposition, a dosage from about 0.1 to about 50 mg/kg will have therapeutic efficacy, with all weights being calculated based upon the weight of the polypeptide, including the cases where a salt is employed. Toxicity concerns at the higher level can restrict intravenous dosages to a lower level such as up to about 10 mg/kg, with all weights being calculated based upon the weight of the polypeptide, including the cases where a salt is employed. A dosage from about 10 mg/kg to about 50 mg/kg can be employed for oral administration. Typically, a dosage from about 0.5 mg/kg to 5 mg/kg can be employed for intramuscular injection. Particular dosages are about 1 µmol/kg to 50 µmol/kg, and more particularly to about 22 µmol/kg and to 33 µmol/kg of the polypeptide for intravenous or oral administration, respectively.

In particular embodiments of the invention, more than one administration (e.g., two, three, four, or more administrations) can be employed over a variety of time intervals (e.g., hourly, daily, weekly, monthly, *etc.)* to achieve therapeutic effects.

The present invention finds use in veterinary and medical applications. Suitable subjects include both avians and mammals, with mammals being preferred. The term "avian" as used herein includes, but is not limited to, chickens, ducks, geese, quail, turkeys, and pheasants. The term "mammal" as used herein includes, but is not limited to, humans, bovines, ovines, caprines, equines, felines, canines, lagomorphs, *etc.* Human subjects include neonates, infants, juveniles, and adults.

The invention will now be described with reference to the following examples. It should be appreciated that these examples are not intended to limit the scope of the claims to the invention but are rather intended to be exemplary of certain embodiments. Any variations in the exemplified methods that occur to the skilled artisan are intended to fall within the scope of the invention.

### EXAMPLES

### Example 1: Novel Immunomodulatory Peptides for Lung Diseases and Disorders

A multi-platform approach was used to study the lung's peptidome for novel immunomodulatory and antiviral therapies, including *in vitro* optimization and *in vitro* and *in vivo* validation of lead compounds in acute and chronic disease models. These studies identified ELD607 as an Orai1/Ca²⁺ inhibitor with broad spectrum efficacy as an immunomodulator in murine infection, allergic, and chronic inflammation models. This study shows that ELD607 inhibits Orai1 in the lung, but not systemically, leading to effective local immunomodulatory action.

The reference ELD100 peptide was incubated overnight in cystic fibrosis (CF) patient sputum, size-fractionated and then analyzed by mass spectrometry without tryptic digest. Three major fragments were found: namely, DITLV, HDIV and NMLIHG, suggesting that ELD100 was cleaved after valines by neutrophil elastase. ELD100 contained 16 residues. A series of peptides were generated including N-terminal, C-terminal and mid-peptide deletions to fully-optimize the peptide's size. This study found that N-terminal deletion of DITLV and removal of all valines was permissible, but that C-terminal deletions, beyond the terminal glycine were not. Using this approach, a library was generated to fully optimize ELD100. The ELD100 parent peptide sequence was extensively modified and superior peptidomimetics were developed that showed enhanced activity with shorter, more potent and more stable peptide sequences. The amino acid sequence modifications included alanine scanning, conservative mutations, shorter sequences and N- and C- terminus modifications (D-ala and Ser additions).

***Alanine scan:*** To identify the key amino acids that retain the thapsigargin-induced inhibition of Ca²⁺ release seen with ELD100, single point changes were introduced to the ELD100 sequence by switching each amino acid to Ala, one at a time. **Table 1** shows the 16 sequences of peptides that resulted from this Ala scan, including the ELDEC ID and the alternative nomenclature. Peptides were pre-incubated for 3 h before adding thapsigargin and measuring fluorescence every 30 s for 7 m in HEK293T cells. The peptides were tested to evaluate the inhibition of Ca²⁺ release upon addition of thapsigargin. **FIG. 4** shows the Ca²⁺ levels for each peptide, including the controls (vehicle and thapsigargin) and the response of ELD 100 for comparison.

Conservative mutations (e.g., replacing Q for N, K for R, etc.) maintain electrostatic charges in residues but alter their size. Conservative mutations were tested to evaluate whether these modifications improved potency of the resultant peptide **(****FIG. 5** **and Table 2).** In addition, by testing shorter sequences, an improved peptide, ELD607, was found that is cheaper to synthesize and more soluble in aqueous solution **(****FIG. 6** and **Table 3).** Amino (N) and carboxy (C) terminus optimization was also performed **(****FIG. 7** and **Table 4).**

Based on these data, five peptides from the "600" series were optimized for resistance to proteolysis. HEK293T cells were plated and incubated for 2 hours with Fluo-4 plus or minus ELD peptides, with fluorescent images taken every 30 seconds for 2-6 minutes at excitation/emission wavelengths of 488/530 nm. **FIG. 8** shows measured cytoplasmic Ca²⁺ after incubation with various ELD peptides, where several peptides including ELD604 (not claimed), 607 (claimed), 100 (reference), and 621 (not claimed) reduced cytoplasmic calcium levels as compared to a control scrambled peptide. Candidate peptides ELD604 (not claimed), 607 (claimed), 601 (not claimed), and 100 (reference) were tested for resistance against protease degradation, wherein the peptides were incubated with neutrophil elastase, treated with the elastase inhibitor sivelestat at various timepoints to halt ongoing reactions, and then analyzed by HPLC **(****FIG. 9****).**

These peptides were not degraded by trypsin. Neutrophil elastase, which is one of the major proteases in the CF lung, was also tested. ELD601 was the shortest peptides generated and lacked valines, which are a substrate for neutrophil elastase. Also made was ELD604 (not claimed), which is ELD601 flanked with D-alanines to prevent exoproteases. Additionally, a series of ELD601-based peptides were generated that incorporated the conservative mutations of **Table 2** that showed increased efficacy including ELD607, where histidine was substituted for an arginine. Despite lacking valines, ELD601 was still degraded by elastase. In contrast, ELD604 was significantly more stable. Surprisingly, despite containing valines and not being flanked by D-alanines, arginine substitution resulted in a significant increase in stability for ELD607 **(****FIG. 9****).**

Of the peptides tested in the mouse model, ELD607 was chosen as a lead peptide following analysis of peptide stability, size and efficacy **(****FIG. 10****).**

***ELD607 is stable in CF sputum.*** CF sputum is highly proteolytic and SPLUNC1 is rapidly degraded in CF sputum. Therefore, it was tested whether ELD607 was stable in CF sputum. ELD607 was incubated with CF sputum supernatant for 6 h at 37°C, and then the reaction was halted by passing the sputum and ELD607 through a 10 kDa cut off spin column at 4°C. Using HPLC, a peak could be detected that corresponded to ELD607 **(****FIG. 11** **panel A).** Neither normal nor CF sputum diminished the area under the curve of this peak, indicating that the peptide was stable over time **(****FIG. 11** **panel B).** To determine whether ELD607 remained functional after exposure to CF sputum, ELD607 that had been incubated for 6 h in either PBS or CF sputum was added to HEK293T cells. Consistent with ELD607 being stable in CF sputum, this peptide retained full functionality and was fully able to inhibit Ca²⁺ influx even after pre-exposure to CF sputum **(****FIG. 11** **panel C).** In contrast, ELD100 (reference), which is not neutrophil elastase resistant, failed to inhibit Ca²⁺ influx after incubation in CF sputum. Importantly, ELD607 was incubated in CF sputum for 6 h before addition to cells, which constitutes a worst-case scenario: in CF patients, ELD607 will likely begin to exert its pharmacological actions much sooner after inhalation.

***ELD607 does not interact with mucus.*** CF airways are characterized by mucus accumulation/dehydration. The predicted pore size of a CF mucin mesh is ~200 nm while ELD607 is estimated to be ≤1 nm so ELD607 should diffuse through mucus at the same rate as it diffuses through water. However, this does not account for possible interactions between ELD607 and gel forming mucins. A previously mucus-coated quartz crystal microbalance with dissipation (QCM-D) was used to detect interactions (changes in dissipation [cross-linking] and frequency [mass]) when peptides are added. Tests were performed to determine whether ELD607 interacted with airway mucus which contained the MUC5AC and MUC5B mucins seen in the CF lung using QCM-D. While the positive control (Poly-L-lysine) bound to mucus and altered cross-linking ad mass, ELD607 did not affect mucus **(****FIG. 12A****).** Other peptides were also tested by QCM-D for frequency **(****FIG. 12B****)** and dissipation **(****FIG. 12C****).**

***ELD607 requires Orail expression to inhibit Ca²⁺ signaling.*** This study found that ELD607 has a similar cell biology to SPLUNC1 and the α6 scaffold peptide (ELD100, reference), and that ELD607 works by inhibiting Orai1. In the absence of extracellular Ca²⁺, thapsigargin stimulates Ca²⁺ release from the ER and ELD607 had no effect on this component **(****FIG. 13****).** While ELD607 reduced Ca²⁺ influx, after shRNA knockdown of Orai1, ELD607 was no longer able to affect Ca²⁺ homeostasis **(****FIG. 13****).** These studies were followed by dose response tests for ELD607 using the Ca²⁺ add back approach shown in **FIG. 13****.** In **FIG. 14****,** the latter Ca²⁺ influx component was shown to be ELD607-sensitive and Orai1 mediated. Using this approach, curves were fitted to the data and the 50% inhibitory concentrations (IC₅₀s) were calculated. The maximal inhibitions are shown in **Table 5.**

Further *in vivo* experiments were performed. Mice were treated intratracheally with 10⁷ colony forming units (CFU) of *P. aeruginosa 1* hour prior to intranasal treatment with 0.51 mg/kg peptide, in a murine model of bacterial pneumonia. Bronchoalveolar lavage (BAL) and organ collection was performed 24 hours following treatment with peptide **(****FIG. 15A****).** Analysis of BAL monocytes **(****FIG. 15B****)** and neutrophils **(****FIG. 15C****)** showed that ELD607 significantly retained monocyte levels and reduced neutrophil levels in the BAL as compared to an uninfected naïve control. Moreover, 24 h after infection, about 10⁹ CFUs could be detected in murine whole lung homogenate. However, ELD607 caused an ~5 log₁₀ reduction in lung bacteria **(****FIG. 15D****).**

Analysis of BAL cytokine levels showed that ELD607 reduced Macrophage Inflammatory Protein 2 (MIP-2), also known as CXCL2, interleukin-17 (IL-17), and keratinocytes-derived chemokine (KC), also known as GROα and CXCL2 **(****FIG. 16A****).** ELD607 also reduced interleukin-1β (IL-1β), tumor necrosis factor-α (TNF-α), and IL-6 **(****FIG. 16B****),** three inflammatory cytokines which have been shown to be elected in COVID-19 patients. Lung histology analysis showed increased inflammation with infection, and showed normalization of alveoli in the infected animals treated with ELD607 **(****FIG. 16C****).** This histology shows that the alveolar/gas exchange regions of murine lungs were protected by ELD607, preventing alveolar damage and thickening that can inhibit gas exchange during infection. Bronchoalveolar lavage (BAL) was collected from mice treated intratracheally with 10⁷ colony forming units (CFU) of *P. aeruginosa* 1 hour prior to intranasal treatment with 0.51 mg/kg peptide, in a murine model of bacterial pneumonia, or vehicle-treated mice. Total protein, neutrophil elastase and lactate dehydrogenase (LDH) levels in the mouse P. *aeruginosa* infection model were also significantly reduced by ELD607 **(****FIG. 16D****).** ELD607 also reduced bacterial counts in the spleen **(****FIG. 16E****),** showing that an inhaled therapeutic can have systemic effects such as reducing sepsis. Similarly, ELD607 also prevented the total weight loss seen with intranasal 10⁷ CFU/ml *P. aeruginosa* infection (FIG. **16F****).** **FIG. 16G** shows histology and graphs of BAL analysis, suggesting that ELD607 reduces Orai1 in murine BAL macrophages. Mice were exposed to vehicle or *P. aeruginosa ±* ELD607 and BAL was obtained and stained with an anti-Orai1 antibody, fluorescent 2° and DAPI. **FIG. 16G** **panel A** shows representative images of macrophages from the different groups as indicated. **FIG. 16G** **panels B and C** show plots showing fluorescent intensities of Orai1 in macrophages and neutrophils, respectively.

In a survival study of mice treated with 10⁹ CFU/ml *P. aeruginosa,* ELD607 also significantly improved survival at 48 hours post-infection **(****FIG. 17A****).** BAL neutrophils were also reduced when ELD607 was added 24 hours after *P. aeruginosa* infection **(****FIG. 17B****).**

ELD607 showed no effect on *P. aeruginosa* growth, indicating that its effects on infection parameters is immunomodulatory. In contrast, penicillin-streptomycin and ciproflaxin controls fully inhibited bacterial growth **(****FIG. 18****).**

***ELD607 reduces neutrophilia and increases lung bacterial clearance in multiple murine bacterial infection models.*** Neutrophilia is a common feature of both Gram-positive and Gram-negative lung infections. Accordingly, further studies were performed with *P*. *aeruginosa, Staphylococcus aureus,* and *Haemophilus influenzae* **(****FIG. 19** **panels B, C, G, H, L,** and **M).** After infection with all three organisms, significant levels of bacteria were detected in the lung and spleen, and the infections increased total BAL cell numbers due to neutrophilia, while relative BAL macrophage numbers declined **(****FIG. 19** **panels A-O).** ELD607, but not ELD100, significantly normalized lung macrophage levels and reduced neutrophilia 24 h after infection. Moreover, 24 h after infection, about 10⁹ CFUs could be detected in murine whole lung homogenate. However, ELD607 caused an ~3-5 log₁₀ reduction in lung bacteria **(****FIG. 19** **panels D** and **I)** and significantly reduced sepsis **(****FIG. 19** **panels E** and **J).** Importantly, while the reference ELD100 scaffold protein has essentially no effect, in all cases, ELD607 significantly improved outcomes.

Additional models were tested such as the house dust mite (HDM) allergic lung inflammation model **(****FIG. 20****);** and SCNN1B-overexpression, wherein mice develop mucus obstruction and spontaneous pulmonary inflammation **(****FIG. 21****).**

These data show that ELD607 inhibits calcium flux upstream of lung cytokines and can reduce lung cytokine levels, eosinophilia, neutrophilia, lung inflammation, lung damage, and sepsis, relevant parameters to many lung diseases and disorders including, but not limited to, allergens (reduced eosinophilia, circulating IgG, and mast cell Ca²⁺, reduced inflammation), bacterial infections (reduced lung cytokines, neutrophilia, lung damage, and sepsis, and improved survival), viral infections (reduced viral replication, predicted reduced cytokines, neutrophilia, lung damage, and improved survival), and mucosal disorders (reduced inflammation and neutrophilia).

To examine Orai1 levels, BAL was then obtained from *P. aeruginosa-infected* mice, immediately cytospun, fixed in PFA and stained with an antibody against Orai1. A significant decrease in Orai1 levels was seen in naive and *P. aeruginosa-infected* mice after ELD607 exposure, indicating successful target engagement.

***ELD607 inhibits viral infections.*** ACE2 protease cDNA was overexpressed in HEK293T cells for 24 hours and then 24 hours later these cells were infected with SARS-CoV-2 S1 Spike protein, DsRed, and VSVG pseudotyped into a lentivirus ± peptide as indicated in **FIG. 22****.** Since this virus expressed DsRed in cells that it infected, DsRed fluorescence could be measured using an imaging plate reader as a marker of infection. ELD607, but not the control scrambled peptide, significantly reduced infection **(****FIG. 22****).** This experiment was repeated with other peptides in the 600 series, showing that ELD604 (not claimed) and ELD621 (not claimed) also inhibited viral replication **(****FIG. 23****).**

***ELD607 normalized leukocyte composition and viral loads in the airways after murine coronavirus infection.*** Coronaviruses are emerging pathogens with severe outcomes in the human population. Mice were infected with 10⁶ PFU mouse hepatitis virus (MHV-A59; a murine coronavirus) intranasally. The mice were then dosed with 1.0 mg/kg ELD607 or vehicle intranasally 4 days p.i. and then 2 days after dosing with ELD607 (6 days p.i.), mice were sacrificed to collect BAL and lung homogenates. ELD607 significantly reduced lung lymphocyte levels (B cells and T cells) and normalized lung monocyte and macrophage levels **(****FIG. 24** **panels A and B).** Moreover, after only one dose of ELD607, viral loads in the lungs and BAL were significantly reduced **(****FIG. 24** **panel C).**
Thus, ELD607 beneficially reduces inflammation in both acute and chronic models. Without wishing to be bound to theory, ELD607 may moderate local inflammation in the lung lumen to reduce neutrophilia and allow macrophages to more efficiently clear bacteria without neutrophil-elastase-mediated lung injury. No evidence of immunocompromization was found, suggesting that ELD607 may be safe to administer to CF patients, who are typically chronically infected with multiple species of bacteria.

### Example 2: Further data regarding ELD607 as a novel peptide-based Orail inhibitor.

HPLC analysis revealed that ELD607 was four times more stable than α6 after exposure to human neutrophil elastase **(****FIG. 25****, panel A).** Endogenous Orai1/store operated Ca²⁺ influx have been extensively studied in HEK293T cells, so this cell type was used to understand ELD607-Orai1 interactions. ELD607 was significantly more potent than α6 at inhibiting store operated Ca²⁺ influx (IC₅₀s were 9 and 124 nM respectively; **FIG. 25****, panel B).** Thapsigargin is a SERCA pump inhibitor that prevents uptake of cytosolic Ca²⁺ into the endoplasmic reticulum. Thapsigargin is commonly used to stimulate Orai1/store operated Ca²⁺ influx. The inhibitory effects of α6 and ELD607 on thapsigargin-induced elevations in cytosolic Ca²⁺ were not additive, indicating that they share the same target **(****FIG. 25****, panel C).** Dynamic light scattering (DLS) was used to evaluate binding of ELD607 to peptides of Orai1 extracellular loops. The intensity weighted harmonic mean particle size (Z-average) significantly increased when Orai1 extracellular peptide 1 (OP1), corresponding to amino acids 106-119 (EVQLDADHDYPPGL; **SEQ ID NO:10)** of Orai1, displayed significant interactions with ELD607 but not scrambled ELD607 peptide control **(****FIG. 25****, panel D).** A peptide of Orai1's second extracellular loop (OP2) corresponding to amino acids 195-234 (CWVKFLPLKKQPGQPRPTSKPPASGAAANVSTSGITPGQA; **SEQ ID NO:11)** of Orai1 did not interact with any of these peptides **(****FIG. 25****, panel E).** Thus, it was concluded that ELD607 interacts with Orai1.

To determine whether ELD607 internalized Orai1, total internal reflection fluorescence (TIRF) microscopy was used to measure Orai1 in the plasma membrane. Neither vehicle control nor scrambled peptide significantly affected Orai1 levels. However, ELD607 reduced Orai1 plasma membrane expression by ~50% **(****FIG. 26****, panels A-B).** Orai1 has been shown to regulate NFAT in multiple cell types, by indirectly dephosphorylating NFAT and allowing it to enter the nucleus where it can act as a transcription factor. We expressed NFAT-GFP in HEK293T cells and evaluated the ability of ELD607 to attenuate NFAT relocation to the nucleus after stimulation of SOCE by confocal microscopy. ELD607 reduced the percentage of NFAT in the nucleus by greater than 50% **(****FIG. 26****, panels C-D).**

***ELD607 is active in ARDS patient immune cells.*** Pulmonary neutrophilic inflammation and increased lung neutrophil elastase levels are common in ARDS patient lungs. We therefore evaluated whether ELD607 remained stable in BAL samples patients with ARDS caused by bacterial pneumonia. Consistent with ELD607 showing increased resistance to neutrophil elastase **(****FIG. 25****, panel A),** ELD607 remained stable over 8 h in ARDS BAL samples at 37°C **(****FIG. 27****, panel A).** Consistent with ELD607 being stable in ARDS BAL, incubation of ELD607 in the ARDS BAL for extended periods did not prevent ELD607 from inhibiting store operated Ca²⁺ influx, when this BAL was placed on HEK293T cells loaded with ELD607 **(****FIG. 27****, panel B).**

We next obtained tracheal aspirates from intubated ARDS/bacterial pneumonia patients. We immediately isolated immune cells from these samples and simultaneously loaded them with the Ca²⁺ dye Fluo-4 and exposed them to vehicle, ELD607 or scrambled peptide for 1 h. Thapsigargin elevated cytosolic Ca²⁺ after vehicle or scrambled peptide exposure, but not after exposure to ELD607 **(****FIG. 27****, panel C).**

We then performed Orai1 immunocytochemistry on paired samples of immune cells of ARDS/bacterial pneumonia patients. Cells derived from each patient were exposed to either vehicle, ELD607, or scrambled ELD607 **(****FIG. 27****, panels D and E).** Again, ELD607, but not vehicle or scrambled peptide exposure, resulted in a significant reduction in Orai 1 levels **(****FIG. 27****, panel E).** Taken together, these data indicate that inhaled ELD607 will be able to inhibit Orai1 in ARDS patient respiratory tracts.

***ELD607 is non-toxic in vitro and in vivo.*** To investigate potential cytotoxic effects of ELD607, HEK294 T cells were exposed to 100 µM ELD607 for 24 h. Cytotoxicity was assessed by exposing cells to calcein to label live cells and fluorescence read by multi-plate reader. ELD607 treated cells were not significantly different to vehicle control indicating no toxic effect **(****FIG. 28****, panel A).** Primary human bronchial epithelial cultures were exposed to 1 mM ELD607 or vehicle control (saline) for 24 h. Cytotoxicity was assessed by confocal microscopy using calcein and propidium iodide to label live and dead cells respectively. ELD607 treated cells were not significantly different to vehicle control indicating no toxic effect **(****FIG. 28****, panels B & C).** Transepithelial resistance was also measured before and after treatment as a measure of epithelial integrity. No differences were found between vehicle and ELD607 treated epithelia **(****FIG. 28****, panel D).**

To assess the pharmacokinetics and safety of ELD607 *in vivo,* mice were treated intratracheally with 10 mg/kg ELD607 daily for 7 days. Mice were sacrificed at 7 days posttreatment. Daily dosing of mice with 10 mg/kg ELD607 for 7 days did not induce any changes in lung histology **(****FIG. 29****, panels A and B)** or body weight (a general indictor of mouse health) **(****FIG. 29****, panel C).**

*ELD607 is retained in the lung lumen.* ELD607-TAMRA or vehicle (saline) was administered to mice intratracheally (0.5 mM, 1 h). Lungs were isolated, fixed, clarified and imaged using light-sheet fluorescence microscopy. ELD607-TAMRA was observed throughout the airway lumen **(****FIG. 30****, panel A)** but not in the interstitia (data representative of 3 individual experiments). We then dosed mice with 10 mg/kg ELD607 and collected BAL and blood. ELD607 levels declined over time in the BAL and plateaued with ~10% of the initial dose detected after 2 h **(****FIG. 30****, panel B).** After 10 mg/kg instillation of ELD607, less than 0.3% of the original dose was detected in the blood at any point in this time frame **(****FIG. 30****, panel C)** suggesting that ELD607 does not have systemic uptake.

*ELD607, but not global Orail inhibition or steroids beneficially reduce pulmonary inflammation after P. aeruginosa infection.* Orai1^{-/-} mice have low survival rates, so we used Orai1^{+/-} mice that have ~70% reduced Ca²⁺ influx (Gwack et al. 2008 Mol cell Biol. 28(17):5209-5222). Orai1^{+/-} mice infected with 10⁷ CFU/mouse *P. aeruginosa* POA1 and treated intranasally 1 h later with 0.5 mg/kg ELD607, were not able to normalize leukocyte levels **(****FIG. 31****, panels A-C).** In contrast, WT littermate controls infected with *P*. *aeruginosa* and treated with ELD607 had reduced normalized leukocytes similar to naïve mice after ELD607 exposure **(****FIG. 31****, panels B and C).** Additionally Orai1^{+/-} mice infected and then treated with ELD607 were not able to clear bacterial burden in the lungs **(****FIG. 31****, panel D).**

We then infected WT mice with *P. aeruginosa* and treated with ELD607, the Orai1 small molecule antagonist YM-58453, dexamethasone or scrambled ELD607. While ELD607 significantly reduced neutrophilia, weight loss and bacterial infections, and increased lung macrophage levels, neither -58453, dexamethasone nor scrambled ELD607 had any significant effects on these parameters **(****FIG. 31****, panels E-H).**

*Repeated ELD607 exposure reduces chronic inflammation in βENaC overexpressing mice.* We added ELD607 to βENaC overexpressing mice, which spontaneously develop chronic infection and neutrophilia and have increased mortality **(****FIG. 32****, panel A).** Daily intranasal addition of ELD607 (0.5 mg/kg) significantly reduced lung neutrophilia, and increased survival **(****FIG. 32****, panel B).**

*ELD607 reduces allergic inflammation.* Mice were exposed to house dust mite allergen for 14 days. 0.5 mg/kg ELD100 (reference) or ELD607 were instilled intranasally for 2 days before sacrifice. ELD607 significantly reduced BAL neutrophilia and eosinophilia, and increased BAL macrophages, in the mouse house dust mite model **(****FIG. 33****, panels A-C).**

*ELD607 reduces inflammation in an acute respiratory distress syndrome (ARDS) model.* Mice were intratracheally to 3 mg/kg *E. coli* lipopolysaccharide (LPS). 0.5 mg/kg ELD607 was instilled intranasally 1 or 6 h later. As a control, 3 mg/kg dexamethasone was given intranasally. ELD607 significantly reduced lung damage caused by LPS **(****FIG. 34****, panels A and B),** reduced weight loss **(****FIG. 34****, panel C)** and attenuated BAL neutrophilia **(****FIG. 34****, panel D).**

### Example 3: ELD607 as a novel peptide-based Orail inhibitor in sepsis.

We injected mice intraperitoneally with *E. coli* LPS, followed by 0.5 mg/kg ELD607 injected intravenously 1 h later. ELD607 administered intravenously significant prevented weight loss **(****FIG. 35****, panel A),** significantly reduced blood neutrophilia **(****FIG. 35****, panel B)** and significantly increased blood macrophages **(****FIG. 35****, panel C).** We next looked for ELD607 stability in human blood and found that it was 100% stable in healthy subject serum for 4 h **(****FIG. 35****, panel D).**

**Table 1. Sequences of ELD100 peptide (reference) undergoing Alanine scan. The alternative names denote the amino acids changed and their position.**

| ELDEC ID | SEQUENCE | SEQ ID NO | ALTERNATIVE NAME |
|---|---|---|---|
| ELD1 (not claimed) | DITLVHDIVNMLIHGL | 12 | α6short |
| ELD31 (not claimed) | AITLVHDIVNMLIHGL | 13 | D1 |
| ELD32 (not claimed) | DATLVHDIVNMLIHGL | 14 | I2 |
| ELD33 (not claimed) | DIALVHDIVNMLIHGL | 15 | T3 |
| ELD34 (not claimed) | DITAVHDIVNMLIHGL | 16 | L4 |
| ELD35 (not claimed) | DITLAHDIVNMLIHGL | 17 | V5 |
| ELD36 (not claimed) | DITLVADIVNMLIHGL | 18 | H6 |
| ELD37 (not claimed) | DITLVHAIVNMLIHGL | 19 | D7 |
| ELD38 (not claimed) | DITLVHDAVNMLIHGL | 20 | I8 |
| ELD39 (not claimed) | DITLVHDIANMLIHGL | 21 | V9 |
| ELD40 (not claimed) | DITLVHDIVAMLIHGL | 22 | N10 |
| ELD41 (not claimed) | DITLVHDIVNALIHGL | 23 | M11 |
| ELD42 (not claimed) | DITLVHDIVNMAIHGL | 24 | L12 |
| ELD43 (not claimed) | DITLVHDIVNMLAHGL | 25 | I13 |
| ELD44 (not claimed) | DITLVHDIVNMLIAGL | 26 | H14 |
| ELD45 (not claimed) | DITLVHDIVNMLIHAL | 27 | G15 |
| ELD46 (not claimed) | DITLVHDIVNMLIHGA | 28 | L16 |

**Table 2. Sequences of ELD100 peptide undergoing conservative mutations. The alternative names denote the amino acids changed and their position.**

| ELDEC ID | SEQUENCE | SEQ ID NO | ALTERNATIVE NAME |
|---|---|---|---|
| ELD121 (not claimed) | EITLVHDIVNMLlHGL-NH₂ | 29 | D1E |
| ELD122 (not claimed) | NITLVHDIVNMLIHGL-NH₂ | 30 | D1N |
| ELD123 (not claimed) | QITLVHDIVNMLIHGL-NH₂ | 31 | D1Q |
| ELD124 (not claimed) | KITLVHDIVNMLIHGL-NH₂ | 32 | D1K |
| ELD125 (not claimed) | DITLVHEIVNMLIHGL-NH₂ | 33 | D7E |
| ELD126 (not claimed) | DITLVHNIVNMLIHGL-NH₂ | 34 | D7N |
| ELD127 (not claimed) | DITLVHQIVNMLIHGL-NH₂ | 35 | D7Q |
| ELD128 (not claimed) | DITLVHKIVNMLIHGL-NH₂ | 36 | D7K |
| ELD129 (not claimed) | DITLVHDLVNMLIHGL-NH₂ | 37 | I8L |
| ELD130 (not claimed) | DITLVHDVVNMLIHGL-NH₂ | 38 | I8V |
| ELD131 (not claimed) | DITLVHDMVNMLIHGL-NH₂ | 39 | I8M |
| ELD132 (not claimed) | DITLVHD-NIe-VNMLIHGL-NH₂ | 40 | I8NIe |
| ELD133 (not claimed) | DITLVHDIVNILIHGL-NH₂ | 41 | M11I |
| ELD134 (not claimed) | DITLVHDIVNLLIHGL-NH₂ | 42 | M11L |
| ELD135 (not claimed) | DITLVHDIVN-NIe-LIHGL-NH₂ | 43 | M11NIe |
| ELD136 (not claimed) | DITLVHDIVNMLMHGL-NH₂ | 44 | I13M |
| ELD137 (not claimed) | DITLVHDIVNMLVHGL-NH₂ | 45 | I13V |
| ELD138 (not claimed) | DITLVHDIVNMLLHGL-NH₂ | 46 | I13L |
| ELD139 (not claimed) | DITLVHDIVNML-NIe-HGL-NH₂ | 47 | I13NIe |
| ELD140 (not claimed) | DITLVHDIVNMLIQGL-NH₂ | 48 | H14Q |
| ELD141 (not claimed) | DITLVHDIVNMLIYGL-NH₂ | 49 | H14Y |
| ELD142 (not claimed) | DITLVHDIVNMLIRGL-NH₂ | 7 | H14R |
| ELD143 (not claimed) | DITLVHDIVNMLINGL-NH₂ | 50 | H14N |
| ELD144 (not claimed) | DITLVHDIVNMLIHPL-NH₂ | 51 | G15P |
| ELD145 (not claimed) | DITLVHDIVNMLIHSL-NH₂ | 52 | G15S |
| ELD146 (not claimed) | DITLVHDIVNMLIHEL-NH₂ | 53 | G15E |
| ELD147 (not claimed) | DITLVHDIVNMLIHNL-NH₂ | 54 | G15N |
| ELD148 (not claimed) | DITLVHDIVNMLIHGM-NH₂ | 55 | L16M |
| ELD149 (not claimed) | DITLVHDIVNMLIHGI-NH₂ | 56 | L16I |
| ELD150 (not claimed) | DITLVHDIVNMLIHGF-NH₂ | 57 | L16F |
| ELD151 (not claimed) | DITLVHDIVNMLIHG-NIe-NH₂ | 58 | NL16NIe |

**Table 3. Sequences of ELD100 undergoing size optimization. The alternative names describe the change introduced compared to the parent peptide.**

| **ELDEC ID** | **SEQUENCE** | **SEQ ID NO** | **ALTERNATIVE NAME** |
|---|---|---|---|
| **ELD3 (not claimed)** | DITLVHDIVNMLIH | 59 | α6s - G |
| **ELD5 (not claimed)** | MINTLDILGHIVHDV | 60 | α6s scrambled |
| **ELD6 (not claimed)** | DITLVH | 61 | α6s-6-C |
| **ELD8 (not claimed)** | DITLVHDI | 62 | α6s-8-C |
| **ELD9 (not claimed)** | DITLVHDIVN | 63 | α6s-10-C |
| **ELD10 (not claimed)** | DITLVHDIVNML | 64 | α6s-12-C |
| **ELD301 (not claimed)** | TLVHDIVNMLIHG | 65 | α6s-13-N |
| **ELD302 (not claimed)** | VHDIVNMLIHG | 3 | α6s-11-N |
| **ELD303 (not claimed)** | DIVNMLIHG | 66 | α6s-9-N |
| **ELD304 (not claimed)** | VNMLIHG | 67 | α6s-7-N |
| **ELD305 (not claimed)** | MLIHG | 68 | α6s-5-N |
| **ELD306 (not claimed)** | TLVHDIVNMLI | 69 | α6s-11-NC |
| **ELD307 (not claimed)** | VHDIVNM | 70 | α6s-7-NC |
| **ELD308 (not claimed)** | DIVNM | 71 | α6s-5-NC |
| **ELD310 (not claimed)** | DITLVHDVNMLIHG | 72 | α6s-14-middle |
| **ELD311 (not claimed)** | DITLVHNMLIHG | 73 | α6s-12-middle |
| **ELD312 (not claimed)** | DITLVMLIHG | 74 | α6s-10-middle |
| **ELD313 (not claimed)** | DITLLIHG | 75 | α6s-8-middle |
| **ELD314 (not claimed)** | DITIHG | 76 | α6s-6-middle |
| **ELD315 (not claimed)** | DITLHDINMLIHG | 77 | α6s-no V |

**Table 4. Sequences of ELD100 undergoing size optimization and capping changes. The alternative names describe the change introduced compared to the parent peptide.**

| **ELDEC ID** | **SEQUENCE** | **SEQ ID NO** | **ALTERNATIVE NAME** |
|---|---|---|---|
| **ELD 601 (not claimed)** | HDINMLIHG | 4 | ELD302 noV |
| **ELD 602 (not claimed)** | a-VHDIVNMLIHG-a | 78 | ELD-302 D-ala |
| **ELD 603 (not claimed)** | a-VHDIVNMLIH-a | 79 | ELD-302 noG, D-ala |
| **ELD 604 (not claimed)** | a-HDIVNMLIH-a | 6 | ELD302 noV, D-ala |
| **ELD 605 (not claimed)** | VHDIVNMLIHP | 80 | ELD144-11 |
| **ELD 606 (not claimed)** | HDINMLIHP | 81 | ELD144-9 |
| **ELD 607 (claimed)** | VHDIVNMLIRG | 8 | ELD142-11 |
| **ELD 608 (not claimed)** | HDINMLIRG | 82 | ELD142-11 noV |
| **ELD 610 (not claimed)** | SVHDIVNMLIHG | 83 | ELD-302 N-Ser |
| **ELD 611 (not claimed)** | VHDIVNMLIHGS | 84 | ELD-302 C-Ser |
| **ELD 612 (not claimed)** | SVHDIVNMLIHGS | 85 | ELD-302 NC-Ser |
| **ELD 620 (not claimed)** | n-HDINMLIHG-n | 9 | ELD-302 no V, C-amidated |
| **ELD 621 (not claimed)** | n-VHDIVNMLIHG-n | 5 | ELD-302 C-amidated |

**Table 5. ELD607 is more potent and efficacious than ELD100 (reference). Data calculated from FIG. 13.**

| | **ELD607 (claimed)** | **ELD100 (reference)** |
|---|---|---|
| IC50 (Molar) | 8.1E-08 | 3.7E-08 |
| Bottom (%) | 37.76 | 57.03 |

**Table 6. Amino acid codon table.**

| **Amino Acid** | | | **Codons** | | | | | |
|---|---|---|---|---|---|---|---|---|
| Alanine | Ala | A | GCA | GCC | GCG | GCT | | |
| Cysteine | Cys | C | TGC | TGT | | | | |
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | ACT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

The foregoing is illustrative of the present invention and is not to be construed as limiting thereof. The invention is defined by the following claims, with equivalents of the claims to be included therein.

## Claims

1. A synthetic polypeptide consisting of:
the amino acid sequence VHDIVNMLIRG **(SEQ ID NO:8);** or
the C-terminally amidated form of amino acid sequence VHDIVNMLIRG **(SEQ ID NO:8).**

2. The synthetic polypeptide of claim 1, consisting of the amino acid sequence VHDIVNMLIRG **(SEQ ID NO:8).**

3. A polynucleotide encoding the synthetic polypeptide of any one of claims 1-2.

4. A vector comprising the polynucleotide of claim 3.

5. A cell comprising the polynucleotide of claim 3 and/or the vector of claim 4.

6. A kit comprising the synthetic polypeptide of any one of claims 1-2, the polynucleotide of claim 3, the vector of claim 4, and/or the cell of claim 5.

7. A composition comprising the synthetic polypeptide of any one of claims 1-2 and a carrier.

8. A pharmaceutical composition comprising the synthetic polypeptide of any one of claims 1-2 and a pharmaceutically acceptable carrier; wherein preferably the pharmaceutical composition further comprises a therapeutic agent selected from the group consisting of dexamethasone, remdesivir, elexacaftor, ivacaftor, lumacaftor, tezacaftor, ibuprofen, acebilustat, lenabasum, levofloxacin, piperacillin-taxobactam, vancomycin, azithromycin, ciprofloxacin, cephalexin, doxycycline hyclate, and any combination thereof.

9. A dosage delivery device comprising the pharmaceutical composition of claim 8; wherein preferably the dosage delivery device is an inhaler.

10. An *in vitro* method of inhibiting calcium influx through a calcium channel, comprising contacting the calcium channel with the synthetic polypeptide of any one of claims 1-2 and/or the pharmaceutical composition of claim 8, thereby inhibiting calcium influx through the calcium channel, wherein the calcium channel is present in an isolated cell.

11. An *in vitro* method of reducing expression of an Orai1 calcium channel, comprising contacting the calcium channel Orai1 with the synthetic polypeptide of any one of claims 1-2 and/or the pharmaceutical composition of claim 8, wherein the calcium channel is present in an isolated cell; wherein preferably the isolated cell is in a cell culture.

12. The synthetic polypeptide of any one of claims 1-2, the polynucleotide of claim 3, the vector of claim 4, and/or the composition of any one of claims 7 and 8; for use in a method of:
(1) inhibiting an immune response in a subject, comprising delivering to the subject said synthetic polypeptide, said polynucleotide, said vector, and/or said composition, thereby inhibiting the immune response; or
(2) inhibiting inflammation in a subject, comprising delivering to the subject said synthetic polypeptide, said polynucleotide, said vector, and/or said composition, thereby inhibiting the inflammation.

13. The synthetic polypeptide, the polynucleotide, the vector, and/or the composition, for use according to claim 12, wherein delivering to the subject said synthetic polypeptide (e.g., said synthetic polypeptide, said polynucleotide, said vector, and/or said composition) comprises delivering the synthetic polypeptide:
(a) directly to the lungs; or
(b) to a target organ system.

14. The synthetic polypeptide of any one of claims 1-2, the polynucleotide of claim 3, the vector of claim 4, and/or the composition of any one of claims 7 and 8; for use in a method of
treating or preventing a disorder, comprising delivering to the airway of a subject a therapeutically or prophylactically effective amount of said synthetic polypeptide, said polynucleotide, said vector, and/or said composition thereby treating or preventing the disorder;
wherein the disorder is asthma, chronic obstructive pulmonary disease ((COPD), an allergy, cystic fibrosis, fibrosis, non-cystic fibrosis bronchiectasis, pneumonia, or an infection.

15. The synthetic polypeptide of any one of claims 1-2, the polynucleotide of claim 3, the vector of claim 4, and/or the composition of any one of claims 7 and 8; for use in a method of treating or preventing a disorder, comprising delivering to a subject a therapeutically or prophylactically effective amount of said synthetic polypeptide, said polynucleotide, said vector, and/or said composition, thereby treating or preventing the disorder; wherein preferably the synthetic polypeptide is delivered systemically to the subject via intravenous delivery, or wherein preferably the synthetic polypeptide, polynucleotide, vector, and/or composition is delivered directly to a target organ system;
wherein the disorder is acute pancreatitis, arthritis, allergy, Bell's Palsy, cardiovascular disease (CVD), dermatomyositis, diabetes, Guillain-Barré Syndrome, inflammatory bowel disease, Lupus, Myasthenia Gravis, psoriasis, reactive arthritis, renal inflammation, rheumatoid arthritis, sarcoidosis, scleroderma, infection, Sjögren's Syndrome, temporal arteritis, Kawasaki disease, or any combination thereof.

## Patentansprüche

1. Synthetisches Polypeptid, bestehend aus:
der Aminosäuresequenz VHDIVNMLIRG (**SEQ ID NO:8**); oder
der C-terminal amidierten Form der Aminosäuresequenz VHDIVNMLIRG (**SEQ ID NO:8**).

2. Synthetisches Polypeptid nach Anspruch 1, bestehend aus der Aminosäuresequenz VHDIVNMLIRG (**SEQ ID NO:8**).

3. Polynukleotid, kodierend für das synthetische Polypeptid nach einem der Ansprüche 1 bis 2.

4. Vektor, umfassend das Polynukleotid nach Anspruch 3.

5. Zelle, umfassend das Polynukleotid nach Anspruch 3 und/oder den Vektor nach Anspruch 4.

6. Kit, umfassend das synthetische Polypeptid nach einem der Ansprüche 1 bis 2, das Polynukleotid nach Anspruch 3, den Vektor nach Anspruch 4, und/oder die Zelle nach Anspruch 5.

7. Zusammensetzung, umfassend das synthetische Polypeptid nach einem der Ansprüche 1 bis 2 und einen Träger.

8. Pharmazeutische Zusammensetzung, umfassend das synthetische Polypeptid nach einem der Ansprüche 1-2 und einen pharmazeutisch akzeptablen Träger; wobei vorzugsweise die pharmazeutische Zusammensetzung ferner einen therapeutischen Wirkstoff umfasst, ausgewählt aus der Gruppe bestehend aus Dexamethason, Remdesivir, Elexacaftor, Ivacaftor, Lumacaftor, Tezacaftor, Ibuprofen, Acebilustat, Lenabasum, Levofloxacin, Piperacillin-Taxobactam, Vancomycin, Azithromycin, Ciprofloxacin, Cephalexin, Doxycyclin-Hyclat und jeder Kombination davon.

9. Verabreichungsvorrichtung, umfassend die pharmazeutische Zusammensetzung nach Anspruch 8; wobei die Verabreichungsvorrichtung vorzugsweise ein Inhalator ist.

10. *In-vitro*-Verfahren zum Inhibieren des Calciumeinstroms durch einen Calciumkanal, umfassend Kontaktieren des Calciumkanals mit dem synthetischen Polypeptid nach einem der Ansprüche 1 bis 2 und/oder der pharmazeutischen Zusammensetzung nach Anspruch 8, dadurch Inhibieren des Calciumeinstroms durch den Calciumkanal, wobei der Calciumkanal in einer isolierten Zelle vorhanden ist.

11. *In-vitro-*Verfahren zum Reduzieren der Expression eines Orai1-Calciumkanals, umfassend Kontaktieren des Orai1-Calciumkanals mit dem synthetischen Polypeptid nach einem der Ansprüche 1 bis 2 und/oder der pharmazeutischen Zusammensetzung nach Anspruch 8, wobei der Calciumkanal in einer isolierten Zelle vorhanden ist; wobei die isolierte Zelle vorzugsweise in einer Zellkultur ist.

12. Synthetisches Polypeptid nach einem der Ansprüche 1 bis 2, Polynukleotid nach Anspruch 3, Vektor nach Anspruch 4 und/oder Zusammensetzung nach einem der Ansprüche 7 und 8; zur Verwendung in einem Verfahren mit:
(1) Inhibieren einer Immunantwort in einem Subjekt, umfassend Verabreichen des synthetischen Polypeptids, des Polynukleotids, des Vektors und/oder der Zusammensetzung an das Subjekt, dadurch Inhibieren der Immunantwort; oder
(2) Inhibieren einer Entzündung in einem Subjekt, umfassend Verabreichen des synthetischen Polypeptids, des Polynukleotids, des Vektors und/oder der Zusammensetzung an das Subjekt, dadurch Inhibieren der Entzündung.

13. Synthetisches Polypeptid, Polynukleotid, Vektor und/oder Zusammensetzung zur Verwendung nach Anspruch 12, wobei Verabreichen des synthetischen Polypeptids (z. B. des synthetischen Polypeptids, des Polynukleotids, des Vektors und/oder der Zusammensetzung) an das Subjekt Verabreichen des synthetischen Polypeptids
(a) direkt in die Lungen; oder
(b) an ein Zielorgansystem umfasst.

14. Synthetisches Polypeptid nach einem der Ansprüche 1 bis 2, Polynukleotid nach Anspruch 3, Vektor nach Anspruch 4 und/oder Zusammensetzung nach einem der Ansprüche 7 und 8;
zur Verwendung in einem Verfahren zum Behandeln oder Verhindern einer Erkrankung, umfassend Verabreichen einer therapeutisch oder prophylaktisch wirksamen Menge des synthetischen Polypeptids, des Polynukleotids, des Vektors und/oder der Zusammensetzung an den Atemweg eines Subjekts, dadurch Behandeln oder Verhindern der Erkrankung;
wobei die Erkrankung Asthma; chronisch obstruktive Lungenerkrankung (COPD), eine Allergie; Mukoviszidose, Fibrose, nichtzystische Bronchiektasie, Pneumonie oder eine Infektion ist.

15. Synthetisches Polypeptid nach einem der Ansprüche 1 bis 2, Polynukleotid nach Anspruch 3, Vektor nach Anspruch 4 und/oder Zusammensetzung nach einem der Ansprüche 7 und 8; zur Verwendung in einem Verfahren zum Behandeln oder Verhindern einer Erkrankung, umfassend Verabreichen einer therapeutisch oder prophylaktisch wirksamen Menge des synthetischen Polypeptids, des Polynukleotids, des Vektors und/oder der Zusammensetzung an ein Subjekt, dadurch Behandeln oder Verhindern der Erkrankung; wobei vorzugsweise das synthetische Polypeptid dem Subjekt systemisch verabreicht wird mittels intravenöser Verabreichung, oder wobei vorzugsweise das synthetische Polypeptid, das Polynukleotid, der Vektor und/oder die Zusammensetzung direkt an ein Zielorgansystem verabreicht wird;
wobei die Erkrankung akute Pankreatitis, Arthritis, Allergie, Bell-Lähmung, Herz-Kreislauf-Erkrankung (HKE), Dermatomyositis, Diabetes, Guillain-Barré-Syndrom, entzündliche Darmerkrankung, Lupus, Myasthenia gravis, Psoriasis, reaktive Arthritis, Nierenentzündung, rheumatoide Arthritis, Sarkoidose, Sklerodermie, Infektion, Sjögren-Syndrom, Temporalisarteriitis, Kawasaki-Syndrom oder eine beliebige Kombination davon ist.

## Revendications

1. Polypeptide synthétique consistant en :
la séquence d'acides aminés VHDIVNMLIRG (SEQ ID NO : 8) ; ou
la forme amidée en position C-terminale de la séquence d'acides aminés VHDIVNMLIRG (SEQ ID NO : 8).

2. Polypeptide synthétique selon la revendication 1, consistant en la séquence d'acides aminés VHDIVNMLIRG (SEQ ID NO : 8).

3. Polynucléotide codant le polypeptide synthétique de l'une quelconque des revendications 1 et 2.

4. Vecteur comprenant le polynucléotide de la revendication 3.

5. Cellule comprenant le polynucléotide de la revendication 3 et/ou le vecteur de la revendication 4.

6. Trousse comprenant le polypeptide synthétique de l'une quelconque des revendications 1 et 2, le polynucléotide de la revendication 3, le vecteur de la revendication 4 et/ou la cellule de la revendication 5.

7. Composition comprenant le polypeptide synthétique de l'une quelconque des revendications 1 et 2 et un véhicule.

8. Composition pharmaceutique comprenant le polypeptide synthétique de l'une quelconque des revendications 1 et 2 et un véhicule pharmaceutiquement acceptable ; laquelle composition pharmaceutique de préférence comprend en outre un agent thérapeutique choisi dans le groupe constitué par la dexaméthasone, le remdésivir, l'élexacaftor, l'ivacaftor, le lumacaftor, le tézacaftor, l'ibuprofène, l'acébilustat, le lénabasum, la lévofloxacine, la pipéracilline-tazobactam, la vancomycine, l'azithromycine, la ciprofloxacine, la céfalexine et la doxycycline hyclate, et l'une quelconque de leurs combinaisons.

9. Dispositif d'administration de dose comprenant la composition pharmaceutique de la revendication 8 ; lequel dispositif d'administration de dose est un inhalateur.

10. Procédé in vitro pour inhiber l'influx de calcium à travers un canal calcique, comprenant la mise en contact du canal calcique avec le polypeptide synthétique de l'une quelconque des revendications 1 et 2 et/ou la composition pharmaceutique de la revendication 8, ce qui ainsi inhibe l'influx de calcium à travers le canal calcique, dans lequel le canal calcique est présent dans une cellule isolée.

11. Procédé *in vitro* pour réduire l'expression d'un canal calcique Orai1, comprenant la mise en contact du canal calcique Orai1 avec le polypeptide synthétique de l'une quelconque des revendications 1 et 2 et/ou la composition pharmaceutique de la revendication 8, dans lequel le canal calcique est présent dans une cellule isolée ; dans lequel de préférence la cellule isolée est en culture cellulaire.

12. Polypeptide synthétique selon l'une quelconque des revendications 1 et 2, polynucléotide selon la revendication 3, vecteur selon la revendication 4, et/ou composition selon l'une quelconque des revendications 7 et 8 ; pour une utilisation dans un procédé pour :
(1) inhiber une réponse immunitaire chez un sujet, comprenant l'administration au sujet dudit polypeptide synthétique, dudit polynucléotide, dudit vecteur et/ou de ladite composition, ce qui ainsi inhibe la réponse immunitaire ; ou
(2) inhiber une inflammation chez un sujet, comprenant l'administration au sujet dudit polypeptide synthétique, dudit polynucléotide, dudit vecteur et/ou de ladite composition, ce qui ainsi inhibe l'inflammation.

13. Polypeptide synthétique, polynucléotide, vecteur et/ou composition pour une utilisation selon la revendication 12, dans lequel l'administration au sujet dudit polypeptide synthétique (par exemple dudit polypeptide synthétique, dudit polynucléotide, dudit vecteur et/ou de ladite composition) comprend l'administration du polypeptide synthétique :
(a) directement aux poumons ; ou
(b) à un système organique cible.

14. Polypeptide synthétique selon l'une quelconque des revendications 1 et 2, polynucléotide selon la revendication 3, vecteur selon la revendication 4, et/ou composition selon l'une quelconque des revendications 7 et 8 ; pour une utilisation dans un procédé pour :
traiter ou prévenir une affection, comprenant l'administration aux voies aériennes d'un sujet d'une quantité efficace du point de vue thérapeutique ou prophylactique dudit polypeptide synthétique, dudit polynucléotide, dudit vecteur et/ou de ladite composition, ce qui ainsi traite ou prévient l'affection ;
dans lequel l'affection est un asthme, une broncho-pneumopathie chronique obstructive (BPCO), une allergie, une mucoviscidose, une fibrose, une bronchiectasie autre que la mucoviscidose, une pneumonie, ou une infection.

15. Polypeptide synthétique selon l'une quelconque des revendications 1 et 2, polynucléotide selon la revendication 3, vecteur selon la revendication 4, et/ou composition selon l'une quelconque des revendications 7 et 8 ; pour une utilisation dans une méthode de traitement ou de prévention d'une affection, comprenant l'administration à un sujet d'une quantité efficace du point de vue thérapeutique ou prophylactique dudit polypeptide synthétique, dudit polynucléotide, dudit vecteur et/ou de ladite composition, ce qui ainsi traite ou prévient l'affection ; dans lequel de préférence le polypeptide synthétique est administré par voie systémique au sujet via administration intraveineuse, ou dans lequel de préférence le polypeptide synthétique, polynucléotide, vecteur et/ou composition est administré directement à un système organique cible ;
dans lequel l'affection est une pancréatite aiguë, une arthrite, une allergie, une paralysie de Bell, une maladie cardiovasculaire (MCV), une dermatomyosite, un diabète, un syndrome de Guillain-Barré, une maladie intestinale inflammatoire, un lupus, une myasthénie grave, un psoriasis, une arthrite réactionnelle, une inflammation rénale, une polyarthrite rhumatoïde, une sarcoïdose, un scléroderme, une infection, un syndrome de Sjögren, une artérite temporale, une maladie de Kawazaki, ou l'une quelconque de leurs combinaisons.
